# EUROPEAN PATENT APPLICATION

(11) **EP 4 428 225 A1**
(43) Date of publication of application: **11.09.2024**
(21) Application number: 23161350.6
(22) Date of filing: 10.03.2023
(51) Int. Cl.: C12N 1/20, C12N 9/04, C12N 9/02, C12P 7/56

(54) **ENGINEERED OBLIGATE FERMENTING BACTERIAFOR THE FERMENTATIVE PRODUCTION OF D-LACTATE**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to genetically engineered bacteria comprising a deletion of *ndh* gene, of one or more *nuo* genes, of *dld* gene, and optionally of *mqo* gene. In particular, the present invention relates to a genetically engineered bacteria comprising a deletion of *ndh* gene, of one or more *nuo* genes, of *dld* gene, optionally of *mqo* gene and further comprising a deletion of one or more genes selected from the group comprising or consisting of *kefF* gene, *wrbA* gene, *yieF* gene, *mdaB* gene, *ygiN gene, putA* gene, *glcDEF* genes,, *dadA* gene, *fadE* gene, *lldD* gene, *glpABC* genes, *glpD, lhgO* gene, *poxB* gene, *sdhABCD* genes and *gpsA* gene.

## Description

The present invention relates to a genetically engineered bacteria comprising a deletion of *ndh* gene, of one or more *nuo* genes, of *dld* gene, and optionally of *mqo* gene. In particular, the present invention relates to a genetically engineered bacteriacomprising a deletion of *ndh* gene, of one or more *nuo* genes, of *dld* gene, optionally of *mqo* gene, and further comprising a deletion of of one or more genes selected from the group comprising or consisting of *poxB* gene, *sdhABCD* genes, *glcDEF* genes, *IhgO* gene, *putA* gene, *lldD* gene, *dadA* gene, *kefF* gene, *mdaB-ygiN* genes, *gIpABC* genes, *gpsA* gene, *wrbA* gene, *yieF* gene, and *fadE* gene.

### Background of the invention

Facultative anaerobe bacteria can grow both under aerobic or anaerobic conditions by using carbohydrates as sole carbon and energy source. In the glycolysis pathway, glucose is transported into the cell by the phosphotransferase system and is phosphorylated to glucose-6-phosphate which is then metabolized to two molecules of pyruvate, two adenosine triphosphates (ATP) and two nicotinamide adenine dinucleotides (NADH). The two NADH generated by the glycolysis pathway are then re-oxidized to NAD⁺.

Under aerobic conditions, thus in the presence of oxygen, the NADH generated during glycolysis is re-oxidized in the aerobic respiratory chain and pyruvate is converted to acetyl-CoA and CO₂ by the pyruvate dehydrogenase complex. Acetyl-CoA is then further processed within the citric acid cycle. The aerobic respiratory chain of *E. coli* involves ubiquinone (Q) mediated electron transfer from dehydrogenases to cytochrome oxidases. NADH dehydrogenase I (NDH-1) and NADH dehydrogenase II (NDH-2) are two NADH dehydrogenases in *E. coli* that oxidize NADH to NAD⁺: Both NADH dehydrogenases donate electrons to the cytochrome oxidases to reduce O₂ to H₂O. The three cytochrome oxidases in *E. coli* are cytochrome *bo* oxidase (CyoABCD), cytochrome *bd*-I oxidase (CydAB) and cytochrome *bd*-II oxidase (AppBC).

The anaerobic respiratory chain of bacteria involves menaquinone (MQ) mediated electron transfer from dehydrogenases to electron accepting terminal reductases. Anaerobic electron donors in include NADH and NADH dehydrogenase I (NDH-1) is also expressed under anaerobic conditions. Anaerobic electron acceptors include fumarate, nitrate, nitrite, trimethylamine-*N*-oxide, and dimethylsulfoxide.

Under anaerobic conditions and in absence of alternative electron acceptors such as fumarate or nitrate, the NADH oxidation steps are accomplished by reduction of several intermediates e.g. pyruvate. A so called "mixed-acid fermentation" can be used to produce lactate, succinate, ethanol, acetate, formate, carbon dioxide, and hydrogen from pyruvate. To achieve a proper fermentation balance, the amount of NADH produced must match the amount of NADH consumed. In general, a mixture of ethanol, lactate and acetate is used, all of which consume different amounts of NADH.

Several engineered *bacteria strains* are known from the prior art that have been modified with the aim of directing the fermentation pathways to a specific fermentation product, e.g. D-lactate, under anaerobic conditions. The usual strategy for providing such engineered strains is to eliminate the fermentation pathways that lead to the production of undesired fermentation products, which is usually achieved by a deletion of the genes encoding the enzymes expressed under anaerobic conditions that catalyze the reactions to the undesired fermentation products. Overexpression of pathways involved in the conversion of the carbon source, e.g. glucose, can also be used to direct the fermentation pathway to a specific fermentation product, e.g. D-lactate. Other engineered bacteria strains are known from the prior art that have been modified to allow the use of alternative carbon sources for the production of specific fermentation end products under anaerobic or micro-aerobic conditions. For example, the use of xylose, sucrose, or glycerol as carbon sources instead of glucose for the production of D-lactate under anaerobic conditions or under micro-aerobic conditions has been described for a few engineered *E. coli* strains. However, the above described *E. coli* strains particularly require strict maintenance of anaerobic or micro-aerobic conditions for the production of D-lactate via fermentation. Thereby, micro-aerobic conditions are even harder to control and adjust in large bioreactors. This represents a major drawback for the use of engineered bacteria strains for the production of D-lactate via fermentation.

The US patent application US 2012/0064581 A1 discloses engineered *E. coli* strains having a deletion of the *cyoABCD* genes, *cydAB* genes and *cbdAD* genes encoding the three cytochrome oxidases in *E. coli* cytochrome *bo* oxidase (cyoABCD), cytochrome *bd*-I oxidase (cydAB) and cytochrome *bd*-II oxidase (appBC). These engineered *E. coli* strains have been further used as platform strains in evolutionary engineering to provide *E. coli* strains with the ability to convert glucose to D-lactate under one or both aerobic and anaerobic conditions. The adaptive evolution of these strains resulted in *E. coli* strains having strong decrease or complete elimination of oxygen uptake. As the oxygen uptake is severely limited, the anaerobic pathways responsible for mixed-acid fermentation is also active under aerobic conditions. These *E. coli* strains are thus suitable for the production of fermentation products, e.g. D-lactate from usual carbon sources such as glucose under "aerobic conditions". However, the use of alternative carbon sources such as glycerol for the production of D-lactate or the exploitation of alternative fermentation pathways is not possible with these *E. coli* strains.

A further drawback associated with the *E. coli* strains of US patent application US 2012/0064581 A1 and with other engineered bacteria from the prior art, such as the bacteria using glycerol as a carbon source instead of glucose for the production of D-lactate under micro-aerobic conditions, is that the initially genetically modified bacteria had to undergo further adaptive evolution to ultimately obtain bacteria with the desired fermentative properties. The use of adaptive evolution has particular drawbacks with regard to the reproducibility and re-engineering of these bacteria. Engineered bacteria that can be obtained by targeted genetic manipulation already having the desired properties without further adaptive evolution are therefore particularly desirable. In addition, it is particularly desirable that engineered bacteria remain stable over time against undesirable evolutionary adaptations and mutations when used in accordance with their intended purpose, e.g. in the production of D-lactate from glucose or alternative carbon sources as glycerol.

In the prior art, the production of chemicals and fuels via microbial fermentation has been largely based on the use of sugars such as glucose, lactose, xylose, etc. as carbon source. Biodiesel is produced by a transesterification reaction using vegetable oils or animal fats and an alcohol, a process that inevitably generates large amounts of glycerol as by-product. It is expected that the production of biodiesel will continue to increase in the future. The further utilization of glycerol produced in biodiesel production is therefore reasonable and desirable, in particular, the conversion of glycerol into higher value products e.g. D-lactate. Glycerol has a high degree of reduction which is advantageous for the microbial production of biofuels and other reduced chemicals compared to glucose. However, only a few bacterial species are known to grow on glycerol under strictly anaerobic conditions without any external electron acceptor. The potential use of glycerol as carbon source in fermentation processes in bacteria is limited by the inability of bacteria to ferment glycerol in the absence of external electron acceptor. It has been extensively investigated whether and under which conditions bacteria can anaerobically grow on glycerol as sole substrate and convert it into ethanol or other valuable products. However, the solutions proposed in the prior art are still insufficient.

Thus, there is a need for engineered bacteria that can be used for the production of specific fermentation products, in particular D-lactate, under aerobic conditions and further for engineered bacteria that allow the conversion of glycerol into higher value products.

It is the objective of the present invention to provide a genetically engineered bacteria for the production of specific fermentation products, in particular D-lactate, under aerobic conditions. A further objective of the present invention is to provide a genetically engineered bacteria for the production of specific fermentation products, in particular D-lactate, from alternative carbon sources such as glycerol. Moreover, it is a further objective of the present invention to provide a genetically engineered bacteria that does not require adaptive evolution techniques for reproduction.

The objective of the present invention is solved by the teaching of the independent claims. Further advantageous features, aspects and details of the invention are evident from the dependent claims, the description, the figures, and the examples of the present application.

### Brief description of the invention

The present invention relates to a genetically engineered obligate fermenting bacteria comprising a deletion of *ndh* gene, of one or more *nuo* genes, of *dld* gene (NNminimal1), and optionally of *mqo* gene (NNminimal2), wherein the one or more *nuo* genes are selected from *nuoA, nuoB, nuoC, nuoD, nuoE, nuoF, nuoG, nuoH, nuoI*, *nuoJ, nuoK, nuoL, nuoM* and *nuoN.* The genetically engineered bacteria of the present invention provides an advantageous platform enabling fermentation processes that can take place in spite of the presence of oxygen, i.e. under aerobic conditions, thereby allowing the use of alternative carbon sources such as glycerol for the production of specific fermentation products, in particular D-lactate.

Moreover, the genetically engineered obligate fermenting NNminimal1 and NNminimal2 of the present invention can be advantageously used as a platform strain for further deletion of one or more additional genes to improve the selective production of specific fermentation end products, in particular of D-lactate, and to prevent the possible production of undesired fermentation by-products. Moreover, it has been found that further deletions of one or more additional genes can be beneficial for provision of a genetically engineered bacteria that is more stable and robust against undesired evolutionary adaptations and mutations.

In preferred embodiments, the genetically engineered bacteria further comprises a deletion of *mqo* gene.

In more preferred embodiments, the genetically engineered bacteria further comprises a deletion of *glpD* gene.

In still more preferred embodiments, the genetically engineered bacteria further comprises a deletion of *poxB* gene and/or a deletion of *sdhABCD* genes.

In further more preferred embodiments, the genetically engineered bacteria further comprises a deletion of one or more genes selected from the group comprising *glcDEF* genes, *of lhgO* gene*,*of *putA* gene, of *IIdD* gene and/or of *dadA* gene.

In further more preferred embodiments, the genetically engineered bacteria further comprises a deletion of one or more genes selected from the group comprising *kefF* gene, *mda8-ygiN* genes, *gIpABC* genes, *gpsA* gene, *wrbA* gene, *yieF* gene, and *fadE* gene.

In preferred embodiments,the one or more *nuo* genes are selected from *nuoE* gene, *nuoF* gene and/or *nuoG* gene, and more preferably the one or more *nuo* genes are *nuoE, nuoF, and nuoG* genes.

Therefore, the present invention preferably relates to a genetically engineered bacteria comprising a deletion of *ndh* gene, of one or more *nuo* genes, of *dld* gene, optionally of *mqo* gene, and/or further comprising a deletion of one or more genes selected from the group comprising or consisting *of poxB* gene, *sdhABCD* genes, *glcDEF* genes, *IhgO* gene, *putA* gene, *IIdD* gene, *dadA* gene, *kefF* gene, *mdaB-ygiN* genes, *gIpABC* genes, *gpsA* gene, *wrbA* gene, *yieF* gene, and *fadE* gene.

A further advantage of the genetically engineered bacteria of the present invention is that it can be easily reproduced by a skilled person by means known in the art and adaptive evolution is not required to obtain the bacteria of the present invention.

In preferred embodiments, the genetically engineered bacteria is a facultativ anaerob bacteria selected from the group comprising *Enterobacteriaceae, Staphylococcaceae, Streptococcaceae, Lactobacillaceae, Vibrionaceae,* and *Pasteurellaceae.*

In further preferred embodiments, the genetically engineered bacteria is an *E*. *coli.*

In still further preferred embodiments, the genetically engineered bacteria is an *E*. *coli.* comprising a deletion of *ndh* gene, of *nuoEFG* genes, of *dld* gene, and optionally a deletion of *mqo* gene .

In further more preferred embodiments, the genetically engineered bacteria is an *E. coli* comprising a deletion of *ndh* gene, of *nuoEFG* genes, of *dld* gene, of *mqo* gene, *poxB* gene and *sdhABCD* genes; and further comprising a deletion of *glcDEF* genes, *of lhgO* gene, of *putA* gene, *of lldD* gene, *of dadA* gene, of *kefF* gene, *of mdaB-ygiN* gene, of *gIpABC* genes, of *gpsA* gene, of *wrbA* gene, *of yieF* gene, *of fadE* gene, and optionally of *g*/*pD.*

In preferred embodiments of the present invention, the *E. coli* is derived from the well-known *E. coli* K-12 MG1655 strain. In even more preferred embodiments, the *E. coli* is an *E. coli* K-12 MG1655 further comprising one or more recombination systems. The one or more recombination systems are preferably selected from an arabinose inducible lambda Red recombineering system and a rhamnose inducible flippase recombinase. Thus, a preferred *E. coli* strain is an *E. coli* K-12 MG1655 strain comprising the genes for arabinose inducible lambda Red recombineering and the genes for rhamnose inducible flippase recombinase.

In contrary to the prior art, the genetically engineered bacteria of the present invention has not been modified to completely eliminate the aerobic respiratory pathway. The aerobic respiratory chain involves ubiquinone (Q) mediated electron transfer to cytochrome oxidases. The cytochrome oxidases *bo*₃, *bd*-I*, bd*-II*,* are the major terminal oxidases in the aerobic respiratory chain and oxidize reduced ubiquinone-8 (UQ-8) and reduce O₂ to two H₂O.

Engineered *E. coli* strains having a deletion of the *cyoABCD* genes, *cydAB* genes and *appBC* genes have been described in US patent application US 2012/0064581 A1. The deletions of these genes prevent the oxygen uptake so that the anaerobic pathways responsible for mixed-acid fermentation are active under aerobic conditions.

However, the genetically engineered bacteria of the present invention have not been modified to exclude the possibility of oxygen uptake, which allows alternative fermentation pathways that make the use of alternative carbon sources such as glycerol for the production of D-lactate by fermentation under aerobic conditions possible in the first place.

Thus, the present invention further relates to a method for producing D-lactate comprising the following steps:
a) providing a genetically engineered bacteria comprising a deletion of *ndh* gene, of one or more *nuo* genes, of *dld* gene, optionally of *mqo* gene, and preferably further comprising a deletion of one or more genes or all of the genes selected from the group comprising or consisting of *poxB* gene, *sdhABCD* genes; *glcDEF* genes, *IhgO* gene, *putA* gene, *IIdD* gene, *dadA* gene, *kefF* gene, *mdaB-ygiN* genes, *gIpABC* genes, *gpsA, wrbA* gene, *yieF* gene, *fadE* gene, and optionally *glpD* gene;
b) providing a culture medium comprising a carbon source;
c) culturing the genetically engineered bacteria under aerobic conditions in the culture medium of b) to produce a culture medium comprising D-lactate;
wherein the carbon source is selected from the group comprising glucose, fructose, xylose, arabinose, galactose, mannose, sucrose, cellobiose, hemicellulose, and glycerol or a combination thereof.

The present invention further relates to a method for producing D-lactate comprising the following steps:
a) providing a genetically engineered bacteria comprising a deletion of *ndh* gene, of one or more *nuo* genes, of *dld* gene, optionally of *mqo* gene, and preferably further comprising a deletion of one or more genes or all of the genes selected from the group comprising or consisting of *poxB* gene, *sdhABCD* genes; *glcDEF* genes, *lhgO* gene, *putA* gene, *lldD* gene, *dadA* gene, *kefF* gene, *mdaB-ygiN* genes, *glpABC* genes, *gpsA, wrbA* gene*, yieF* gene, *fadE* gene, and a *glpD* deletion;
b) providing a culture medium comprising a carbon source; and
c) culturing the genetically engineered bacteriaunder aerobic conditions in the culture medium of b) to produce a culture medium comprising D-lactate,
wherein the carbon source is selected from the group comprising glucose, fructose, xylose, arabinose, galactose, mannose, sucrose, cellobiose, and hemicellulose, or a combination thereof.

The present invention further relates to a method for producing D-lactate comprising the following steps:
a) providing a genetically engineered bacteria comprising a deletion of *ndh* gene, of one or more *nuo* genes, of *dld* gene, optionally of *mqo* gene, and preferably further comprising a deletion of one or more genes or all of the genes selected from the group comprising or consisting of *poxB* gene, *sdhABCD* genes; *glcDEF* genes, *IhgO* gene, *putA* gene, *IIdD* gene, *dadA* gene, *kefF* gene, *mdaB-ygiN* genes, *gIpABC* genes, *gpsA, wrbA* gene*, yieF* gene, *and fadE* gene;
b) providing a culture medium comprising a carbon source; and
c) culturing the genetically engineered bacteria in the culture medium of b) to produce a culture medium comprising D-lactate,

wherein the carbon source is glycerol, and
wherein the genetically engineered *E. coli* strain does not comprise a *glpD* deletion.

The present invention further relates to a method for producing D-lactate comprising the following steps:
a) providing a genetically engineered bacteria comprising a deletion of *ndh* gene, of one or more *nuo* genes, of *dld* gene, optionally of *mqo* gene, and preferably further comprising a deletion of one or more genes or all of the genes selected from the group comprising or consisting of *poxB* gene, *sdhABCD* genes; *glcDEF* genes, *IhgO* gene, *putA* gene, *IIdD* gene, *dadA* gene, *kefF* gene, *mdaB-ygiN* genes, *gIpABC* genes, *gpsA, wrbA* gene*, yieF* gene, *fadE* gene, and a *glpD* deletion;
b) providing a culture medium comprising glucose; and
c) culturing the genetically engineered *E. coli* strain in the culture medium of b) to produce a culture medium comprising D-lactate.

The present invention further relates to a method for producing D-lactate comprising the following steps:
a) providing a genetically engineered bacteria comprising a deletion of *ndh* gene, of one or more *nuo* genes, of *dld* gene, optionally of *mqo* gene, and preferably further comprising a deletion of one or more genes or all of the genes selected from the group comprising or consisting of *poxB* gene, *sdhABCD* genes; *glcDEF* genes, *IhgO* gene, *putA* gene, *IIdD* gene, *dadA* gene, *kefF* gene, *mdaB-ygiN* genes, *gIpABC* genes, *gpsA, wrbA* gene*, yieF* gene, *and fadE* gene;
b) providing a culture medium comprising glycerol; and
c) culturing the genetically engineered bacteria in the culture medium of b) to produce a culture medium comprising D-lactate.

Preferably, the method for producing D-lactate further comprises the steps:
d) collecting the culture medium comprising D-lactate; and
e) isolating D-lactate from the culture solution.

In a preferred embodiment of the method for producing D-lactate described above, the genetically engineered bacteria is a bacteria selected from the group comprising *Enterobacteriaceae, Staphylococcaceae, Streptococcaceae, Lactobacillaceae, Vibrionaceae,* and *Pasteurellaceae.*

In a more preferred embodiment of the method for producing D-lactate described above, the genetically engineered bacteria is an *E. coli.*

### Description of the invention

It has been found that a genetically engineered bacteria comprising a deletion of *ndh* gene, of one or more *nuo* genes, of *dld* gene and optionally of *mqo* gene enables fermentation processes that can take place in spite of the presence of oxygen, i.e. under aerobic conditions, further allowing the use of alternative carbon sources such as glycerol for the production of specific fermentation products, in particular D-lactate.

Moreover, it has been found that a genetically engineered bacteria comprising a deletion of *ndh* gene, of one or more *nuo* genes, and of *dld* gene, and further comprising a deletion of *mqo* gene and a deletion of one or more genes selected from the group comprising or consisting of genes for NADPH dehydrogenase activity, such as *kefF, wrba, yieF, mdaB-ygiN* genes, reactions of NADH dehydrogenase bypassing activities: *putA, glcDEF, dadA, fadE, glpD, lldD, glpABC, lhgO, gpsA,* and quinone reducing reactions from central metabolism: *poxB, sdhABCD,* improved selective production of specific fermentation end products, e.g. in particular D-lactate, and is stable and robust against undesired evolutionary adaptations and mutations.

Therefore, the target genes are grouped on the basis of the activity of encoded proteins as follows:
**Group A:** NADH-dehydrogenase(s): *ndh, nuoEFG;*
**Group B:** NADPH dehydrogenase activity described: *kefF, wrbA, yieF, mdaB, ygiN;*
**Group C:** quinone reducing reactions from central metabolism: *poxB, sdhABCD;*
**Group D:** reactions of NADH dehydrogenase bypassing activities: *dld, mqo, putA, glcDEF, dadA, fadE, glpD, IIdD, glpABC, IhgO.*

The genetically engineered bacteria of the present invention have not been modified to exclude the possibility of oxygen uptake, which allows alternative fermentation pathways that make the use of alternative carbon sources such as glycerol for the production of D-lactate by fermentation under aerobic conditions possible in the first place.

Therefore, the present invention relates to a genetically engineered bacteria comprising a deletion of *ndh* gene, of one or more *nuo* genes, and of *dld* gene.

Preferably, the present invention relates to a genetically engineered bacteria comprising a deletion of *ndh* gene, of one or more *nuo* genes, of *dld* gene, and optionally of *mqo* gene, and further comprising a deletion of one or more genes selected from the group comprising or consisting of *poxB* gene, *sdhABCD* genes, *glcDEF* genes, *IhgO* gene, *putA* gene, *IIdD* gene, *dadA* gene, *kefF* gene, *mdaB-ygiN* genes, *glpABC* genes, *glpD* gene, *gpsA* gene, *wrbA* gene, *yieF* gene, and *fadE* gene

Particularly preferably, the present invention relates to a genetically engineered bacteria comprising a deletion of *ndh* gene, of one or more *nuo* genes, of *dld* gene, and of *mqo* gene, and further comprising a deletion of all of the genes of the group consisting of: *poxB* gene, *sdhABCD* genes, *glcDEF* genes, *lhgO* gene, *putA* gene, *lldD* gene, *dadA* gene, *kefF* gene, *mdaB-ygiN* genes, *glpABC* genes, *glpD* gene, *gpsA* gene, *wrbA* gene, *yieF* gene, and *fadE* gene.

### Preferred gene deletions for the genetically engineered E. coli strain

The term **"NADH dehydrogenase",** as used herein, relates to an enzyme of EC class 1.6.5.11.that catalyzes the chemical reaction

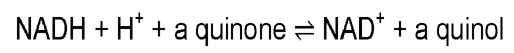

The term ***"nuo* gene"**, as used herein, relates to a gene that encodes one subunit of the protein complex NADH:ubiquinone oxidoreductase I (NuoABCDEFGHIJKLMN, NDH-1). The *nuo* genes in *E. coli* include 14 genes: *nuoABCDEFGHIJKLMN* genes, encoding 13 - 14 subunits, nuoC and nuoD are thought to form one subunit. Thus, NADH:ubiquinone oxidoreductase I of E. *coli* is made up of 13 - 14 different subunits, the NuoA to NuoN subunits. This organization in 13-14 different subunits is conserved in several other bacteria, including *Salmonella typhimurium, Paracoccus denitrificans, Rhodobacter capsulatus,* and *Thermus thermophilus.* The *nuoA* gene encodes the subunit NuoA of NDH-1. The *nuoB* gene encodes the subunit NuoB of NDH-1. The *nuoC* gene encodes the subunit NuoC of NDH-1 and the *nuoD* gene encodes the subunit NuoD of NDH-1, or *nuoC* and *nuoD* genes encode the *nuoCD* subunit. The *nuoE* gene encodes the subunit NuoE of NDH-1. The *nuoF* gene encodes the subunit NuoF of NDH-1. The *nuoG* gene encodes the subunit NuoG of NDH-1. The *nuoH* gene encodes the subunit NuoH of NDH-1. The *nuoI* gene encodes the subunit Nuol of NDH-1. The *nuoJ* gene encodes the subunit NuoJ of NDH-1. The *nuoK* gene encodes the subunit NuoK of NDH-1. The *nuoL* gene encodes the subunit NuoL of NDH-1. The *nuoM* gene encodes the subunit NuoM of NDH-1. The *nuoN* gene encodes the subunit NuoN of NDH-1.NADH:ubiquinone oxidoreductase I (NDH-1) of *E. coli* consists of three components: a soluble fragment composed of the NuoE, F and G subunits, an amphipathic connecting fragment composed of the NuoB, CD and I subunits, and a hydrophobic membrane fragment composed of the NuoA, H, J, K, L, M and N subunits. Thereby, the soluble NADH:ubiquinone oxidoreductase I fragment consisting of the NuoE, NuoF and NuoG subunits represents the electron input part of NADH:ubiquinone oxidoreductase I.

The term **"NADH:ubiquinone oxidoreductase I"** (NDH-1), as used herein, relates to a protein complex of the respiratory chains of many organisms from bacteria to humans. NADH:ubiquinone oxidoreductase I belongs to EC class 7.1.1.2. In *E. coli,* NADH:ubiquinone oxidoreductase I (NDH-1) is one of two distinct NADH dehydrogenases that catalyze the transfer of electrons from NADH to the quinone pool in the cytoplasmic membrane and is able to generate a proton electrochemical gradient.

The term ***"ndh* gene"**, as used herein, relates to a gene that encodes the enzyme NADH:quinone oxidoreductase II.

The term **"NADH:quinone oxidoreductase II"** (NDH-2), as used herein, relates to an alternative, non-proton pumping NADH:quinone oxidoreductase that delivers electrons to the respiratory chain by oxidation of NADH and reduction of quinones. Thus, NADH:quinone oxidoreductase II is one of two distinct NADH dehydrogenases that catalyze the transfer of electrons from NADH to the quinone pool in the cytoplasmic membrane but does not generate an electrochemical gradient as NDH-1 does. NADH:quinone oxidoreductase II. NADH:ubiquinone oxidoreductase II belongs to EC class 1.6.5.9.

The term "**KefF**", (synonym: *yabF*), as used herein, relates to the Glutathione-regulated potassium-efflux system ancillary protein. KefF is an activator of potassium transport mediated by the KefC antiporter. KefF also has enzymatic activity as a quinone oxidoreductase, thereby reducing the redox toxicity of electrophilic quinones.

The term **"wrbA gene",** as used herein, relates to a gene that encodes a NAD(P)H dehydrogenase (quinone).

The term **"WrbA",** as used herein, relates to a protein that has NAD(P)H:quinone oxidoreductase activity. WrbA is related to the flavodoxin family of proteins. Unlike the flavodoxins, WrbA does not have a stabilized semiquinone state. It rapidly takes up two electrons, generating the fully reduced form.

The term **"*yieF* gene"** (synonym: chrR), as used herein, relates to a gene that encodes a quinone reductase.

The term **"YieF"**, as used herein, relates to a flavoprotein containing the FMN cofactor that belongs to the flavodoxin superfamily of enzymes. YieF was shown to possess quinone reductase activity which may guard against oxidative stress by preventing redox cycling of quinones which would otherwise generate ROS, and by maintaining a pool of reduced quinone in the cell that is able to quench ROS directly. The quinone reductase activity of YieF is considered as the primary biological role of this enzyme.

The term ***"ygiN* gene"**, as used herein, relates to a gene in *E. coli* that encodes the probable quinol monooxygenase YgiN. The ygiN gene may be transcribed in an operon together with *mdaB,* indicated as *mdaB-ygiN.*

The term **"YgiN",** as used herein, relates to a protein "probable quinol monooxygenase" that is able to re-oxidize menadiol that has been reduced by "MdaB quinone reductase" in vitro. The two enzymes "probable quinol monooxygenase" and "MdaB quinone reductase" may form a quinone redox cycle. The biological role of a quinone redox cycle is considered to maintain an intracellular pool of menadione and ubiquinone using a catalytic mechanism that avoids the formation of a semiquinone intermediate, and to act as a quinone buffer.

The term *"****mdaB* gene",** as used herein, relates to a gene that encodes the NADPH:quinone oxidoreductase MdaB.

The term **"MdaB",** as used herein, relates to the protein "MdaB quinone reductase" that is specific for NADPH and is most active with quinone derivatives and ferricyanide as electron acceptors. *In vitro,* YgiN is able to reoxidize menadiol that has been reduced by MdaB quinone reductase; the two enzymes may form a quinone redox cycle.

The term ***"mdaB-ygiN* genes"** or ***"mdaB-ygiN* operon",** as used herein, relates to the genes or operon encoding a NADPH:quinone oxidoreductase and a "probable quinol monooxygenase" presumably forming a quinone redox cycle.

The term **"*putA* gene"** (synonym: poaA), as used herein, relates to a gene in that encodes the bifunctional protein PutA.

The term **"PutA",** as used herein, relates to the bifunctional protein PutA that is involved in step 1 and 2 of the sub-pathway that synthesizes L-glutamate from L-proline. The bifunctional protein PutA includes the domains proline dehydrogenase and pyrroline-5-carboxylate dehydrogenase. PutA is a flavoprotein with mutually exclusive functions as a transcriptional repressor and membrane-associated enzyme. The switch between the two activities is due to conformational changes triggered by the redox state of FAD. In the presence of L-proline, PutA is associated with the cytoplasmic membrane and acts a bifunctional enzyme catalyzing both reactions of the proline degradation pathway: the oxidation of proline by proline dehydrogenase and subsequent oxidation to glutamate by pyrroline-5-carboxylate (P5C) dehydrogenase. The kinetics of the coupled reaction is best described by substrate channeling. In the absence of proline, PutA is cytoplasmic and functions as a transcriptional repressor of the put regulon. Proline dehydrogenase activity requires the presence of an electron acceptor. The reaction is split into a reductive half reaction, the reduction of the FAD cofactor by oxidation of proline, and an oxidative half reaction, the re-oxidation of reduced FADH₂ by transfer of electrons to the quinone pool in the cytoplasmic membrane.

The term **"*glcDEF* genes"** *(*synonyms: *gox, yghM),* as used herein, relates to the genes *glcD, glE,* and *glcF* that encode the subunits of the enzyme glycolate oxidase.

More in particular, the term **"GlcDEF",** as used herein, relates to the three subunits of the enzyme glycolate oxidase GlcD, GlcE and GlcF. GlcDEF is a component of a complex that catalyzes the oxidation of glycolate to glyoxylate. GlcDEF is required by bacteria to grow on glycolate as a sole carbon source. The ability to oxidize D-lactate has been described in connection with GlcDEF. GlcDEF does not link directly to O₂.

The term "***dld* gene**", as used herein, relates to a gene that encodes the enzyme quinone-dependent D-lactate dehydrogenase.

The term **"Dld",** as used herein, relates to the enzyme quinone-dependent D_lactate dehydrogenase that is a FAD-dependent peripheral membrane dehydrogenase catalyzing the oxidation of D-lactate to pyruvate. D-lactate dehydrogenase (Dld) is a respiratory enzyme; electrons derived from D-lactate oxidation are transferred to the membrane soluble quinone pool.

The term ***"mqo* gene"** (synonym: yojH), as used herein, relates to a gene that encodes the enzyme malate:quinone oxidoreductase (Mqo).

The term **"Mqo"**, as used herein, relates to the enzyme the malate:quinone oxidoreductase which is a membrane-associated enzyme that catalyzes the oxidation of malate to oxaloacetate. Electrons are likely donated to the electron transfer chain at the quinone level.

The term *"****dadA* gene"** (synonym: dadR), as used herein, relates to a gene that encodes the enzyme D-amino acid dehydrogenase (DadA).

The term **"DadA",** as used herein, relates to the enzyme D-amino acid dehydrogenase. *E* L- and D-alanine can be used by bacteria as the sole source of carbon, nitrogen and energy. D-amino acid dehydrogenase is the second enzyme of the L-alanine degradation I pathway. The enzyme has broad substrate specificity; it catalyzes the oxidative deamination of many D-amino acids, although D-alanine is the best substrate. The enzyme is membrane-associated and linked to the respiratory chain.

The term ***"fadE* gene"** (synonym: yafH), as used herein, relates to a gene that encodes the enzyme Acyl-coenzyme A dehydrogenase (FadE). fadE mutants are unable to utilize oleate and other fatty acids as the sole source of carbon.

The term **"FadE",** as used herein, relates to the enzyme Acyl-coenzyme A dehydrogenase that catalyzes the first step in the degradation of fatty acids via the β-oxidation cycle.

The term ***"gipd* gene"** (synonym: yafH), as used herein, relates to a gene that encodes aerobic glycerol-3-phosphate dehydrogenase (GlpD).

The term **"glpD",** as used herein, relates to the enzyme aerobic glycerol-3-phosphate dehydrogenase that catalyzes the oxidation of sn-glycerol 3-phosphate to dihydroxyacetone phosphate. GlpD is a respiratory enzyme and shuttles electrons via a non-covalently bound FAD cofactor to reduce ubiquinone. Glycerol 3-phosphate is an obligatory intermediate in phospholipid biosynthesis and thus glpD expression is regulated to ensure that phospholipid biosynthesis is maintained while the energy needs of the cell are met. GlpD is required for aerobic growth with glycerol or glycerol 3-phosphate.

A *glpD* deletion is not essential to metabolize a particular carbon source, such as a corbon source selected from the group comprising glucose, fructose, xylose, arabinose, galactose, mannose, sucrose, cellobiose, and hemicellulose or a combination thereof. However, a *glpD* deletion seems to induce higher stability in bacteria when growing on a carbon source selected from the group comprising glucose, fructose, xylose, arabinose, galactose, mannose, sucrose, cellobiose, and hemicellulose. In contrast, a *glpD* deletion seems to be necessary in *E. coli* strain growing on a carbon source being glycerol.

The term "***lldD* gene**" *(*synonym: *lctD*), as used herein, relates to a gene that encodes L-lactate dehydrogenase.

The term **"LldD",** as used herein, relates to the enzyme L-lactate dehydrogenase that is an FMN-dependent membrane-associated dehydrogenase. It functions in aerobic respiration and also has a role in anaerobic nitrate respiration. L-lactate dehydrogenase is associated with the inner membrane. LldD is one of three lactate dehydrogenase enzymes which interconvert pyruvate and lactate. The other two enzymes are specific for D-lactate: the soluble LdhA, an NAD-linked fermentative enzyme, and Dld, a membrane-associated respiratory enzyme. L-lactate dehydrogenase is induced by aerobic growth on L-lactate and L-fucose and in the presence of lactate under nitrate, fumarate and TMAO respiration conditions.

The term **"*glpABC* genes",** as used herein, relates to the genes that encode anaerobic glycerol-3-phosphate dehydrogenase (GlpABC).

The term **"GlpABC",** as used herein, relates to the enzyme anaerobic glycerol-3-phosphate dehydrogenase that catalyzes the oxidation of glycerol-3-phosphate to dihyroxyacetone phosphate. GlpABC is a respiratory enzyme; anaerobic growth of bacteria on glycerol and fumarate induces expression of an anaerobic glycerol-3-phosphate dehydrogenase and fumarate reductase and is associated with proton translocation and the generation of a proton motive force. The GlpABC enzyme is loosely associated with the cell membrane. Bacteria, such as *E. coli* K-12, contain two glycerol-3-phosphate dehydrogenases encoded by the *gIpABC* and *glpD* genes. GlpABC is required for anaerobic growth with glycerol or glycerol-3-phosphate and fumarate as the terminal electron acceptor while GlpD is required for aerobic growth with glycerol (or glycerol-3-phosphate).

The term "***lhgO* gene**" *(*synonym: *lhgD*), as used herein, relates to a gene that encodes-2-hydroxyglutarate dehydrogenase (LhgO).

The term **"LhgO",** as used herein, relates to the enzyme L-2-hydroxyglutarate dehydrogenase which is an electron transport chain-coupled dehydrogenase that feeds electrons from the reaction into the membrane quinone pool. LhgD contains an FAD cofactor which is not covalently attached, and whose reduction potential is relatively high at -25 mV. LhgD is associated with the cytoplasmic membrane, and its activity is only found in the membrane fraction.

The term ***"poxB* gene",** as used herein, relates to a gene that encodes pyruvate dehydrogenase [ubiquinone] (PoxB).

The term **"PoxB",** as used herein, relates to the enzyme pyruvate dehydrogenase [ubiquinone] which is a peripheral membrane enzyme that catalyzes the oxidative decarboxylation of pyruvate to form acetate and CO₂. The reaction is coupled to the electron transport chain via ubiquinone. Metabolism of pyruvate by pyruvate oxidase is less efficient than the route via pyruvate dehydrogenase (PDH); however, the pyruvate oxidase route is important for wild-type growth efficiency and responsible for a significant amount of pyruvate metabolism under aerobic conditions.

The term ***"sdhABCD* genes",** as used herein, relates to the genes encoding succinate dehydrogenase or succinate:quinone oxidoreductase (SdhABCD).

The term **"SdhABCD",** as used herein, relates to the enzyme succinate:quinone oxidoreductase that catalyzes the oxidation of succinate to fumarate concomitant with the reduction of ubiquinone to ubiquinol. SdhABCD plays an important role in cellular metabolism and directly connects the TCA cycle with the respiratory electron transport chain. As part of the TCA cycle succinate is oxidized to fumarate by SdhABCD and electrons are transferred to the membrane quinone pool for entry into the electron transport chain. SdhABCD does not contribute to the proton motive force; the sites of quinol reduction and succinate oxidation are both located on the cytoplasmic side of the membrane and there is no separation of charge across the membrane during catalysis. SdhCDAB is a membrane bound heterotetramer. Subunits SdhA and SdhB are hydrophilic and attached to the cytoplasmic surface of the plasma membrane via interactions with the two hydrophobic integral membrane subunits, SdhC and SdhD. SdhA contains the FAD cofactor and the dicarboxylic acid binding site. Electrons from the oxidation of succinate are transferred through the iron-sulphur protein, SdhB, to a quinone binding site located at the interface of the SdhB, SdhC and SdhD subunits. The SdhC and SdhD subunits each contain three transmembrane helices and anchor the complex to the membrane. A single heme b556 cofactor bridges the SdhC and SdhD subunits.

The term *"****gpsA* gene",** as used herein, relates to a gene that encodes the enzyme glycerol-3-phosphate dehydrogenase (GpsA).

The term **"GpsA",** as used herein, relates to the enzyme glycerol-3-phosphate dehydrogenase catalyzes the NAD(P)H-dependent reduction of the glycolytic intermediate dihydroxyacetone-phosphate to produce glycerol-3-phosphate, a precursor for the biosynthesis of phospholipids.

The term "***ldhA* gene**", as used herein, relates to a gene that encodes the enzyme D-lactate dehydrogenase (LdhA).

The term **"LdhA",** as used herein, relates to the enzyme D-lactate dehydrogenase. LdhA is a soluble NAD-linked lactate dehydrogenase (LDH) that is specific for the production of D-lactate.

The term ***"ubiC* gene"** as used herein, relates to a gene that encodes the enzyme chorismate pyruvate-lyase (UbiC).

The term **"UbiC",** as used herein, relates to the enzyme chorismate pyruvate-lyase that catalyzes the first reaction step in the biosynthesis of ubiquinone which involves the formation of 4-hydroxybenzoate from chorismate. *ubiC* mutants are deficient in the formation of ubiquinone and are characterized by the inability to grow aerobically on oxidizable substrates such as succinate.

The term "***ubiA* gene**" as used herein, relates to a gene that encodes the enzyme 4-hydroxybenzoate octaprenyltransferase (UbiA).

The term **"UbiA",** as used herein, relates to the enzyme 4-hydroxybenzoate octaprenyltransferase that catalyzes the second reaction step in the biosynthesis of ubiquinone which involves the prenylation of 4-hydroxybenzoate with an all-trans polyprenyl group.

The term ***"ubiCA* genes"** or ***"ubiCA* operon"** as used herein, relates to the operon encoding the enzymes chorismate lyase and 4-hydroxybenzoate transferase for the first two committed steps of ubiquinone (also named Coenzyme Q, or Coenzyme Q10, UQ) biosynthesis.

For the provision of a genetically engineered bacteria enabling fermentation processes that can take place in the presence of oxygen, i.e. under aerobic conditions, and further allowing the use of alternative carbon sources such as glycerol for the production of specific fermentation products, in particular D-lactate, alternative solutions to the complete disruption of the aerobic respiratory chain, e.g. deletion of the a deletion of the *cyoABCD* genes, *cydAB* genes and *cbdAD* genes encoding the three cytochrome oxidases in *E. coli* cytochrome *bo* oxidase (cyoABCD), cytochrome *bd*-I oxidase (cydAB) and cytochrome *bd*-II oxidase (appBC) have been envisaged.

The inventor of the present invention suggested that the controlled regulation of oxygen uptake without completely preventing it will have an advantageous effect generating bacteria with a fermentative phenotype which can be used for the production of specific fermentation end products by fermentation under aerobic conditions. Thus, the deletion of the *nuo* genes and *ndh* gene were initially targeted in bacteria t, as the NADH:ubiquinone oxidoreductase I (NDH-1, NuoABCDEFGHIJKLMN) and NADH:quinone oxidoreductase II (NDH-2) are the two distinct NADH dehydrogenases that catalyze the oxidation of NADH to NAD and mainly catalyze the transfer of electrons from NADH to the quinone pool in the cytoplasmic membrane.

Thus, a genetically engineered bacteria having a deletion of *ndh* gene and of one or more *nuo* genes was obtained in the first step. In particular, a deletion of *ndh* gene and of *nuoEFG* genes was performed in an *E. coli* K-12 strain MG1655. The so obtained genetically engineered Δ*nuo,* Δ*nuoEFG E. coli* strain did not show a reduction in biomass production compared to the wild type. Thus, for provision of an obligate fermenting phenotype of genetically engineered bacteria that allows fermentative process under aerobic condition further manipulations were required.

In the next step, it was considered that the prevention of ubiquinone (Q) mediated electron transfer from the NADH dehydrogenases to cytochrome oxidases by disrupting the synthesis of ubiquinone (Q) itself would lead to the desired obligate fermenting phenotype of genetically engineered bacteria. Thus, in order to prevent ubiquinone (Q) mediated electron transfer from the NADH dehydrogenases to cytochrome oxidases a deletion of *ubi* genes for prevention of the biosynthesis of ubiquinone was carried out.

Thus, a further genetically engineered bacterium was obtained by deletion of *ubiA* gene and *ubiC* gene. A genetically engineered *ΔubiCA E. coli* strain was thereby obtained. The inventor of the present invention has found that the *ΔubiCA E. coli* strain mainly produced acetate along D-lactate on glucose or glycerol. This indicates that the Δ*ubiCA E. coli* is still able to get rid of some electrons from NADH to respiration. Thus, for provision of an obligate fermenting *E. coli* strain further manipulation was necessary.

Thus, a further genetically engineered bacterium was obtained by a deletion of *ndh* gene, of one or more *nuo* genes, of *ubiA* gene and of *ubiC* gene. A genetically engineered Δ*nuo,* Δ *nuoEFG,* Δ*ubiCA E. coli* strain having a deletion of *ndh* gene and *nuoEFG* genes and a further deletion of *ubiCA* gene was thereby obtained. In this way indeed a first obligate fermenting *E. coli* strain could be obtained and was named **"NNQ"** strain. The inventor of the present invention has found that the *nuoEFG, ndh* and *ubiCA* deleted *E. coli* strain showed strong reduction of production of acetate and selective production of D-lactate on glucose. However, the production of acetate was increased on glycerol in comparison to glucose. Thus, this strain still produced an undesired amount of acetate along D-lactate when cultured on glycerol as carbon source.

Moreover, the genetically engineered Δ*nuo,* Δ*nuoEFG,* Δ*ubiCA E. coli* strains have shown further disadvantages as a loss of function mutation in *ubiE* could be identified with the *nuo, ndh* and *ubiCA* deletion *E. coli* strain in further experiments. It is known from the prior art that a mutation in *ubiE* leads to accumulation of demethylmenaquinone. Therefore, a demethylmenaquinone-dependent NADH-dehydrogenase-like cycle between pyruvate and lactate was observed for this Δ*nuo*, Δ*nuoEFG*, *ΔubiCA E. coli* strain strain, with NADH consumption over LdhA and demethylmenaquinone-dependent lactate dehydrogenase over Did. The demethylmenaquinone is used in the mini-cycle between pyruvate and lactate via the D -lactate dehydrogenase (Dld) and the pyruvate is thereby reduced to lactate by NADH and subsequently re-oxidized to pyruvate, wherein a quinone is reduced and the electrons get into the respiratory chain. The effects of the described loss of function mutation in *ubiE* correspond to the effect of a further gene deletion of the *ubiE* gene, resulting both in activation of the mini-cycle between pyruvate and lactate. Thus, a further deletion of the *ubiE* does not provide a solution to the observed accumulation of demethylmenaquinone following the mutation in *ubiE.* The acetate production observed in the *ΔubiCA E. coli* strain and the Δ*nuo*, Δ*nuoEFG*, Δ*ubiCA E. coli* strain indicates that these *E. coli* strains may be still capable of electron transfer from NADH into the respiratory chain or may be able to use another electron acceptor instead of ubiquinone e.g. through PoxB activity for acetate production. In consequence, the deletions of one or more *ubi* genes have been found to produce *E. coli* strains that are unsteady against undesired evolutionary adaptations and mutations.

It has been described in the prior art that the deletion of the three cytochrome oxidases, i.e. a deletion of the *cyoABCD* genes, *cydAB* genes and *cbdAD* genes and ygiN gene results in utilization of anaerobic respiration pathways even under aerobic growth conditions and in a shift in the quinone pool from ubiquinones to menaquinones. The disruption of the biosynthesis of ubiquinone in *E. coli* by deletion of one or more *ubi* genes leads also to a shift in the quinone pool from ubiquinones to menaquinones, as the ubiquinones are not available anymore. However, as described above, it has been observed that in presence of oxygen the *E. coli* strains having a deletion of one or more *ubi* genes try to compensate for the lack of ubiquinone in other ways, e.g. evolutionary adaptions and mutations.

Thus, for provision of the desired genetically engineered bacteria a different strategy was necessary. The inventors of the present invention have first identified further suitable enzymes that catalyze quinone-dependent reactions for further gene deletions in Δ*nuo*, Δ*nuoEFG E. coli* strain. The following genes have been selected for deletions in the Δ*nuo*, Δ *nuoEFG*bacteria: *kefF* gene, *wrbA* gene, *yieF* gene, *mdaB-ygiN* genes, genes *putA* gene, *glcDEF* genes, *dld* gene, *mqo* gene, *dadA* gene, *fadE* gene, *lldD* gene, *gIpABC* genes, *glpD, lhgO* gene, *poxB* gene, *sdhABCD* genes and *gpsA* gene.

It has been found that a genetically engineered bacteria comprising a deletion of *ndh* gene, of one or more *nuo* genes, and of *dld* gene comprises the minimum required of gene deletions to enable fermentation processes that can take place in the presence of oxygen, i.e. under aerobic conditions, allowing the use of alternative carbon sources such as glycerol for the production of specific fermentation products, in particular D-lactate. The genetically engineered bacteria according to the present invention particularly do not have the disadvantages of instability of the genetically engineered bacteria *ΔubiCA* and Δ*nuo*, Δ*nuoEFG*, Δ*ubiCA.*

Thus, the present invention relates to a genetically engineered bacteria comprising a deletion of *ndh* gene, a deletion of one or more *nuo* genes, and a deletion of *dld* gene, and optionally a deletion of *mqo* gene, wherein the one or more *nuo* genes are selected from *nuoA, nuoB, nuoC, nuoD, nuoE, nuoF, nuoG, nuoH, nuoI*, *nuoJ, nuoK, nuoL, nuoM* and *nuoN.*

Preferably, the present invention relates to a genetically engineered bacteria comprising a deletion of *ndh* gene, a deletion of one or more *nuo* genes, a deletion of *dld* gene, and optionally a deletion of *mqo* gene, wherein the one or more *nuo* genes are selected from *nuoE, nuoF* and *nuoG.*

However, genetically engineered bacteria comprising a deletion of *ndh* gene, a deletion of one or more *nuo* genes, a deletion of *dld* gene, a deletion of *mqo* gene, and a deletion of *nuoEFG* genes are also in accordance with the present invention.

With other words, the genetically engineered bacteria preferably comprises a deletion of *ndh* gene, a deletion of *nuoE,* of *nuoF* and of *nuoG* genes, a deletion of *dld* gene, and optionally a deletion of *mqo* gene. Thus, the genetically engineered bacteria preferably comprises a deletion of *ndh* gene, a deletion of *nuoEFG* genes, a deletion of *dld* gene, and optionally a deletion of *mqo* gene. More preferably, the genetically engineered bacteria preferably comprises a deletion of *ndh* gene, a deletion of *nuoEFG* genes, a deletion of *dld* gene, and a deletion of *mqo* gene.

In a further embodiment the genetically engineered bacteria according to the present invention may further comprise a deletion of *nuoEFG* together with deletion of the *nuo* genes *nuoA, nuoB, nuoCD, nuoH, nuoI*, *nuoJ, nuoK, nuoL, nuoM* and *nuoN.*

Thus, in further embodiments of the present invention the genetically engineered bacteria may comprise a deletion of *ndh* gene, a deletion of *nuoEFG* genes, a deletion of *dld* gene, optionally a deletion of *mqo* gene, and further comprising deletion of one or more *nuo* genes selected from the group *nuoA, nuoB, nuoCD, nuoH, nuoI*, *nuoJ, nuoK, nuoL, nuoM* and *nuoN.*

Thus, the present invention further relates to a genetically engineered bacteria comprising a deletion of *ndh* gene, a deletion of one or more *nuo* genes, a deletion of *dld* gene, and a deletion of *glpD* gene, wherein the one or more *nuo* genes are selected from *nuoA, nuoB, nuoC, nuoD, nuoE, nuoF, nuoG, nuoH, nuoI*, *nuoJ, nuoK, nuoL, nuoM* and *nuoN.*

Thus, the present invention further relates to a genetically engineered bacteria comprising a deletion of *ndh* gene, a deletion of one or more *nuo* genes, a deletion of *dld* gene, a deletion of *mqo* gene, and a deletion of *glpD* gene, wherein the one or more *nuo* genes are selected from *nuoA, nuoB, nuoC, nuoD, nuoE, nuoF, nuoG, nuoH, nuoI*, *nuoJ, nuoK, nuoL, nuoM* and *nuoN.*

Preferably, the genetically engineered bacteria preferably comprises a deletion of *ndh* gene, a deletion of one or more *nuo* genes, a deletion of *dld* gene, a deletion of *mqo* gene, a deletion of *glpD* gene, and a deletion of at least one of the *nuo*-genes selected from *nuoE, nuoF* and *nuoG.*

In preferred embodiment, the genetically engineered bacteria comprises a deletion of *ndh* gene, a deletion of one or more *nuo* genes, a deletion of *dld* gene, a deletion of *mqo* gene, a deletion of *glpD* gene, wherein the one or more *nuo* genes are *nuoE, nuoF* and *nuoG.* With other words, the genetically engineered bacteria preferably comprises a deletion of *ndh* gene, a deletion of *nuoE,* of *nuoF* and of *nuoG* genes, a deletion of *dld* gene, optionally a deletion of *mqo* gene, and a deletion of *glpD* gene. Thus, the genetically engineered bacteria preferably comprises a deletion of *ndh* gene, a deletion of *nuoEFG* genes, a deletion of *dld* gene, optionally a deletion of *mqo* gene, and a deletion of *glpD* gene. More preferably, the genetically engineered bacteria preferably comprises a deletion of *ndh* gene, a deletion of *nuoEFG* genes, a deletion of *dld* gene, a deletion of *mqo* gene, and a deletion of *glpD* gene.

In a further embodiment the genetically engineered bacteria according to the present invention may further comprise a deletion of *glpD* gene, a deletion of *nuoEFG* together with deletion of the *nuo* genes *nuoA, nuoB, nuoCD, nuoH, nuoI*, *nuoJ, nuoK, nuoL, nuoM* and *nuoN.*

Thus, in further embodiments of the present invention the genetically engineered bacteria may comprise a deletion of *ndh* gene, a deletion of *nuoEFG* genes, a deletion of *dld* gene, optionally a deletion of *mqo* gene, a deletion of *glpD* gene and further comprising deletion of one or more *nuo* genes selected from the group *nuoA, nuoB, nuoCD, nuoH, nuoI*, *nuoJ, nuoK, nuoL, nuoM* and *nuoN.*

In a preferred embodiment, the genetically engineered bacteria may further comprise a deletion of *poxB,* and *sdh* genes enconding for enzymes whose activities are directly coupled to central metabolism.

Preferably, the present invention preferably relates to a genetically engineered bacteria comprising a deletion of *ndh* gene, a deletion of one or more *nuo* genes, a deletion of *dld* gene, a deletion of *mqo* gene; and further comprising a deletion of *poxB* gene and/or *of sdhABCD* gene.

More preferably, the present invention preferably relates to a genetically engineered bacteria comprising a deletion of *ndh* gene, a deletion of one or more *nuo* genes, a deletion of *dld* gene, a deletion of *mqo* gene; and further comprising a deletion of *poxB* gene and/or of *sdhABCD* genes, wherein the one or more *nuo* genes are selected from *nuoE, nuoF* and *nuoG.* With other words, the present invention preferably relates to a genetically engineered bacteria comprising a deletion of *ndh* gene, a deletion of *nuoEFG* genes, a deletion of *dld* gene, a deletion of *mqo* gene; and further comprising a deletion of *poxB* gene and/or *of sdhABCD* gene.

Preferably, the present invention preferably relates to a genetically engineered bacterium comprising a deletion of *ndh* gene, a deletion of one or more *nuo* genes, a deletion of *dld* gene, and a deletion of *mqo* gene.

More preferably, the present invention preferably relates to a genetically engineered bacteria comprising a deletion of *ndh* gene, a deletion of one or more *nuo* genes, a deletion of *dld* gene, and further comprising a deletion of *mqo* gene, wherein the one or more *nuo* genes are selected from *nuoE, nuoF* and *nuoG.* With other words, the present invention preferably relates to a genetically engineered bacteria comprising a deletion of *ndh* gene, a deletion of *nuoEFG* genes, a deletion of *dld* gene, and further comprising a deletion of *mqo* gene.

Preferably, the present invention preferably relates to a genetically engineered bacteria comprising a deletion of *ndh* gene, a deletion of one or more *nuo* genes, a deletion of *dld* gene, and further comprising a deletion *of poxB* gene.

More preferably, the present invention preferably relates to a genetically engineered bacteria comprising a deletion of *ndh* gene, a deletion of one or more *nuo* genes, a deletion of *dld* gene, and further comprising a deletion of *poxB* gene, wherein the one or more *nuo* genes are selected from *nuoE, nuoF* and *nuoG.* With other words, the present invention preferably relates to a genetically engineered bacteria comprising a deletion of *ndh* gene, a deletion of *nuoEFG* genes, a deletion of *dld* gene, and further comprising a deletion of *poxB* gene.

Preferably, the present invention preferably relates to a genetically engineered bacteria comprising a deletion of *ndh* gene, a deletion of one or more *nuo* genes, a deletion of *dld* gene, and further comprising a deletion of *sdhABCD* genes.

More preferably, the present invention preferably relates to a genetically engineered bacteria comprising a deletion of *ndh* gene, a deletion of one or more *nuo* genes, a deletion of *dld* gene, and further comprising a deletion of *sdhABCD* genes, wherein the one or more *nuo* genes are selected from *nuoE, nuoF* and *nuoG.* With other words, the present invention preferably relates to a genetically engineered bacteria comprising a deletion of *ndh* gene, a deletion of *nuoEFG* genes, a deletion of *dld* gene, and further comprising a deletion of *sdhABCD* genes.

Preferably, the present invention preferably relates to a genetically engineered bacteria comprising a deletion of *ndh* gene, a deletion of one or more *nuo* genes, a deletion of *dld* gene, and further comprising a deletion of *mqo* gene and *poxB* gene.

More preferably, the present invention preferably relates to a genetically engineered bacteria comprising a deletion of *ndh* gene, a deletion of one or more *nuo* genes, a deletion of *dld* gene, and further comprising a deletion of *mqo* gene and *poxB* gene, wherein the one or more *nuo* genes are selected from *nuoE, nuoF* and *nuoG.* With other words, the present invention preferably relates to a genetically engineered *bacteria* comprising a deletion of *ndh* gene, a deletion of *nuoEFG* genes, a deletion of *dld* gene, and further comprising a deletion of *mqo* gene and *poxB* gene.

Preferably, the present invention preferably relates to a genetically engineered *bacteria* comprising a deletion of *ndh* gene, a deletion of one or more *nuo* genes, a deletion of *dld* gene, and further comprising a deletion of *mqo* gene and *sdhABCD* gene.

More preferably, the present invention preferably relates to a genetically engineered *bacteria* comprising a deletion of *ndh* gene, a deletion of one or more *nuo* genes, a deletion of *dld* gene, and further comprising a deletion of *mqo* gene and *sdhABCD* genes, wherein the one or more *nuo* genes are selected from *nuoE, nuoF* and *nuoG.* With other words, the present invention preferably relates to a genetically engineered *bacteria* comprising a deletion of *ndh* gene, a deletion of *nuoEFG* genes, a deletion of *dld* gene, and further comprising a deletion of *mqo* gene and or *sdhABCD* gene.

Preferably, the present invention preferably relates to a genetically engineered *bacteria* comprising a deletion of *ndh* gene, a deletion of one or more *nuo* genes, a deletion of *dld* gene, and further comprising a deletion of *poxB* gene and *sdhABCD* gene.

More preferably, the present invention preferably relates to a genetically engineered *bacteria* comprising a deletion of *ndh* gene, a deletion of one or more *nuo* genes, a deletion of *dld* gene, and further comprising a deletion of *poxB* gene and *sdhABCD* genes, wherein the one or more *nuo* genes are selected from *nuoE, nuoF* and *nuoG.* With other words, the present invention preferably relates to a genetically engineered *bacteria* comprising a deletion of *ndh* gene, a deletion of *nuoEFG* genes, a deletion of *dld* gene, and further comprising a deletion of *poxB* gene and *sdhABCD* gene.

Preferably, the present invention preferably relates to a genetically engineered *bacteria* comprising a deletion of *ndh* gene, a deletion of one or more *nuo* genes, a deletion of *dld* gene, and further comprising a deletion of *mqo* gene, *poxB* gene and *sdhABCD* genes.

More preferably, the present invention preferably relates to a genetically engineered *bacteria* comprising a deletion of *ndh* gene, a deletion of one or more *nuo* genes, a deletion of *dld* gene, and further comprising a deletion of *mqo* gene, *poxB* gene and *sdhABCD* genes, wherein the one or more *nuo* genes are selected from *nuoE, nuoF* and *nuoG.* With other words, the present invention preferably relates to a genetically engineered *bacteria* comprising a deletion of *ndh* gene, a deletion of *nuoEFG* genes, a deletion of *dld* gene, and further comprising a deletion of *mqo* gene, *poxB* gene and *sdhABCD* genes.

In preferred embodiments, the genetically engineered bacteria may further comprise a deletion of *glcDEF* genes, *lhgO* gene and/or *putA* gene.

Preferably, the present invention preferably relates to a genetically engineered bacteria comprising a deletion of *ndh* gene, a deletion of one or more *nuo* genes, a deletion of *dld* gene, and further comprising a deletion of *glcDEF* genes, *IhgO* gene and/or *putA* gene.

More preferably, the present invention preferably relates to a genetically engineered bacteria comprising a deletion of *ndh* gene, a deletion of one or more *nuo* genes, a deletion of *dld* gene, and further comprising a deletion of *glcDEF* genes, *IhgO* gene and/or *putA* gene, wherein the one or more *nuo* genes are selected from *nuoE, nuoF* and *nuoG.* With other words, the present invention preferably relates to a genetically engineered bacteria comprising a deletion of *ndh* gene, a deletion of *nuoEFG* genes, a deletion of *dld* gene, and further comprising a deletion of *glcDEF* genes, *IhgO* gene and/or *putA* gene.

Preferably, the present invention preferably relates to a genetically engineered bacteria comprising a deletion of *ndh* gene, a deletion of one or more *nuo* genes, a deletion of *dld* gene, and further comprising a deletion of *glcDEF* genes.

More preferably, the present invention preferably relates to a genetically engineered bacteria comprising a deletion of *ndh* gene, a deletion of one or more *nuo* genes, a deletion of *dld* gene, and further comprising a deletion of *glcDEF* gen, wherein the one or more *nuo* genes are selected from *nuoE, nuoF* and *nuoG.* With other words, the present invention preferably relates to a genetically engineered bacteria comprising a deletion of *ndh* gene, a deletion of *nuoEFG* genes, a deletion of *dld* gene, and further comprising a deletion of *glcDEF* gene.

Preferably, the present invention preferably relates to a genetically engineered bacteria comprising a deletion of *ndh* gene, a deletion of one or more *nuo* genes, a deletion of *dld* gene, and further comprising a deletion of *lhgO* gene.

More preferably, the present invention preferably relates to a genetically engineered bacteria comprising a deletion of *ndh* gene, a deletion of one or more *nuo* genes, a deletion of *dld* gene, and further comprising a deletion of *IhgO* gene, wherein the one or more *nuo* genes are selected from *nuoE, nuoF* and *nuoG.* With other words, the present invention preferably relates to a genetically engineered bacteria comprising a deletion of *ndh* gene, a deletion of *nuoEFG* genes, a deletion of *dld* gene, and further comprising a deletion of *lhgO* gene.

Preferably, the present invention preferably relates to a genetically engineered bacteria comprising a deletion of *ndh* gene, a deletion of one or more *nuo* genes, a deletion of *dld* gene, and further comprising a deletion of *putA* gene.

More preferably, the present invention preferably relates to a genetically engineered bacteria comprising a deletion of *ndh* gene, a deletion of one or more *nuo* genes, a deletion of *dld* gene, and further comprising a deletion of *putA* gene, wherein the one or more *nuo* genes are selected from *nuoE, nuoF* and *nuoG.* With other words, the present invention preferably relates to a genetically engineered bacteria comprising a deletion of *ndh* gene, a deletion of *nuoEFG* genes, a deletion of *dld* gene, and further comprising a deletion of *putA* gene.

Preferably, the present invention preferably relates to a genetically engineered bacteria comprising a deletion of *ndh* gene, a deletion of one or more *nuo* genes, a deletion of *dld* gene, and further comprising a deletion of *glcDEF* genes and *IhgO* gene.

More preferably, the present invention preferably relates to a genetically engineered bacteria comprising a deletion of *ndh* gene, a deletion of one or more *nuo* genes, a deletion of *dld* gene, and further comprising a deletion of *glcDEF* genes *and IhgO* gene, wherein the one or more *nuo* genes are selected from *nuoE, nuoF* and *nuoG.* With other words, the present invention preferably relates to genetically engineered bacteria comprising a deletion of *ndh* gene, a deletion of *nuoEFG* genes, a deletion of *dld* gene, and further comprising a deletion of *glcDEF* genes *and lhgO* gene.

Preferably, the present invention preferably relates to a genetically engineered bacterium comprising a deletion of *ndh* gene, a deletion of one or more *nuo* genes, a deletion of *dld* gene, and further comprising a deletion of *glcDEF* genes and *putA* gene.

More preferably, the present invention preferably relates to a genetically engineered bacteria comprising a deletion of *ndh* gene, a deletion of one or more *nuo* genes, a deletion of *dld* gene, and further comprising a deletion of *glcDEF* genes and *putA* gene, wherein the one or more *nuo* genes are selected from *nuoE, nuoF* and *nuoG.* With other words, the present invention preferably relates to genetically engineered bacteria comprising a deletion of *ndh* gene, a deletion of *nuoEFG* genes, a deletion of *dld* gene, and further comprising a deletion of *glcDEF* genes and *putA* gene.

Preferably, the present invention preferably relates to a genetically engineered bacteria comprising a deletion of *ndh* gene, a deletion of one or more *nuo* genes, a deletion of *dld* gene; and further comprising a deletion of *IhgO* gene and *putA* gene.

More preferably, the present invention preferably relates to a genetically engineered bacteria comprising a deletion of *ndh* gene, a deletion of one or more *nuo* genes, a deletion of *dld* gene; and further comprising a deletion of *IhgO* gene and *putA* gene, wherein the one or more *nuo* genes are selected from *nuoE, nuoF* and *nuoG.* With other words, the present invention preferably relates to a genetically engineered bacteria comprising a deletion of *ndh* gene, a deletion of *nuoEFG* genes, a deletion of *dld* gene; and further comprising a deletion of *IhgO* gene and *putA* gene.

Preferably, the present invention preferably relates to a genetically engineered bacteria comprising a deletion of *ndh* gene, a deletion of one or more *nuo* genes, a deletion of *dld* gene; and further comprising a deletion of *glcDEF* genes, *IhgO* gene and *putA* gene.

More preferably, the present invention preferably relates to a genetically engineered bacteria comprising a deletion of *ndh* gene, a deletion of one or more *nuo* genes, a deletion of *dld* gene; and further comprising a deletion of *glcDEF* genes, *IhgO* gene and *putA* gene, wherein the one or more *nuo* genes are selected from *nuoE, nuoF* and *nuoG.* With other words, the present invention preferably relates to a genetically engineered bacteria comprising a deletion of *ndh* gene, a deletion of *nuoEFG* genes, a deletion of *dld* gene; and further comprising a deletion of *glcDEF* genes, *IhgO* gene and *putA* gene.

Even more preferably, the present invention relates to a genetically engineered bacteria comprising a deletion of *ndh* gene, a deletion of one or more *nuo* genes, a deletion of *dld* gene; and a deletion of *mqo* gene, *poxB* gene and/or *sdhABCD* genes, and further comprising a deletion of *glcDEF* genes, *IhgO* gene and/or *putA* gene.

Even more preferably, the present invention relates to a genetically engineered bacteria comprising a deletion of *ndh* gene, a deletion of *nuoEFG* genes, a deletion of *dld* gene; a deletion of *mqo* gene, *poxB* gene and/or *sdhABCD* genes, and further comprising a deletion of *glcDEF* genes, *IhgO* gene and/or *putA* gene.

Even more preferably, the present invention relates to a genetically engineered bacteria comprising a deletion of *ndh* gene, a deletion of one or more *nuo* genes, a deletion of *dld* gene; a deletion of *mqo* gene, and further comprising a deletion of *glcDEF* genes, *IhgO* gene and/or *putA* gene.

Even more preferably, the present invention relates to a genetically engineered bacteria comprising a deletion of *ndh* gene, a deletion of *nuoEFG* genes, a deletion of *dld* gene; a deletion of *mqo* gene, and further comprising a deletion of *glcDEF* genes*, lhgO* gene and/or *putA* gene.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *glcDEF* genes, *lhgO* gene and/or *putA* gene.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *glcDEF* genes, *IhgO* gene and/or *putA* gene.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *glcDEF* genes, *IhgO* gene and/or *putA* gene.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *glcDEF* genes, *IhgO* gene and/or *putA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, and further comprising a deletion of *glcDEF* genes, *IhgO* gene and/or *putA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *glcDEF* genes, *IhgO* gene and/or *putA* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *glcDEF* genes, *lhgO* gene and/or *putA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *glcDEF* genes, *lhgO* gene and/or *putA* gene.

Even more preferably, the present invention relates to a genetically engineered bacteria comprising a deletion of *ndh* gene, a deletion of one or more *nuo* genes, a deletion of *dld* gene; a deletion of *poxB* gene, a deletion of *sdhABCD* genes, and further comprising a deletion of *glcDEF* genes, *lhgO* gene and/or *putA* gene.

Even more preferably, the present invention relates to a genetically engineered bacteria comprising a deletion of *ndh* gene, a deletion of *nuoEFG* genes, a deletion of *dld* gene; a deletion of *poxB* gene, a deletion of *sdhABCD* genes, and further comprising a deletion of *glcDEF* genes, *IhgO* gene and/or *putA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *glcDEF* genes, *IhgO* gene and/or *putA* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *glcDEF* genes, *IhgO* gene and/or *putA* gene.

Preferably, the present invention relates to a genetically engineered bacteria comprising a deletion of *ndh* gene, a deletion of one or more *nuo* genes, a deletion of *dld* gene; a deletion of *mqo* gene, and further comprising a deletion of *putA* gene.

Even more preferably, the present invention relates to a genetically engineered bacteria comprising a deletion of *ndh* gene, a deletion of *nuoEFG* genes, a deletion of *dld* gene; a deletion of *mqo* gene, and further comprising a deletion of *putA* gene.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *putA* gene.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *putA* gene.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *putA* gene.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *putA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, and further comprising a deletion of *putA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *putA* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *putA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *putA* gene.

Even more preferably, the present invention relates to a genetically engineered bacteria comprising a deletion of *ndh* gene, a deletion of one or more *nuo* genes, a deletion of *dld* gene; a deletion of *poxB* gene, a deletion of *sdhABCD* genes, and further comprising a deletion of *putA* gene.

Even more preferably, the present invention relates to a genetically engineered bacteria comprising a deletion of *ndh* gene, a deletion of *nuoEFG* genes, a deletion of *dld* gene; a deletion of *poxB* gene, a deletion of *sdhABCD* genes, and further comprising a deletion of *putA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *putA* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *putA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *glcDEF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *glcDEF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *IhgO* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *lhgO* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *glcDEF* genes *and IhgO* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *glcDEF* genes *and IhgO* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *glcDEF* genes and *putA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *glcDEF* genes and *putA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *IhgO* gene and *putA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *IhgO* gene and *putA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *glcDEF* genes, *IhgO* gene and *putA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *glcDEF* genes, *IhgO* gene and *putA* gene.

In preferred embodiments, the genetically engineered bacteria may further comprise a deletion of *IIdD* gene and/or *dadA* gene.

Preferably, the present invention preferably relates to a genetically engineered bacteria comprising a deletion of *ndh* gene, a deletion of one or more *nuo* genes, a deletion of *dld* gene; and further comprising a deletion of *IIdD* gene and/or *dadA* gene..

More preferably, the present invention preferably relates to a genetically engineered bacteria comprising a deletion of *ndh* gene, a deletion of *nuoEFG* genes, a deletion of *dld* gene; and further comprising a deletion of *IIdD* gene and/or *dadA* gene.

Preferably, the present invention preferably relates to a genetically engineered bacteria comprising a deletion of *ndh* gene, a deletion of one or more *nuo* genes, a deletion of *dld* gene; and further comprising a deletion of *IIdD* gene.

More preferably, the present invention preferably relates to a genetically engineered bacteria comprising a deletion of *ndh* gene, a deletion of *nuoEFG* genes, a deletion of *dld* gene; and further comprising a deletion of *lldD* gene.

Preferably, the present invention preferably relates to a genetically engineered bacteria comprising a deletion of *ndh* gene, a deletion of one or more *nuo* genes, a deletion of *dld* gene; and further comprising a deletion of *dadA* gene.

More preferably, the present invention preferably relates to a genetically engineered bacteria comprising a deletion of *ndh* gene, a deletion of *nuoEFG* genes, a deletion of *dld* gene; and further comprising a deletion of *dadA* gene.

Preferably, the present invention preferably relates to a genetically engineered bacteria comprising a deletion of *ndh* gene, a deletion of one or more *nuo* genes, a deletion of *dld* gene; and further comprising a deletion of *lldD* gene and *dadA* gene.

More preferably, the present invention preferably relates to a genetically engineered bacteria comprising a deletion of *ndh* gene, a deletion of *nuoEFG* genes, a deletion of *dld* gene; and further comprising a deletion of *lldD* gene and *dadA* gene.

Even more preferably, the present invention relates to a genetically engineered bacteria comprising a deletion of *ndh* gene, a deletion of one or more *nuo* genes, a deletion of *dld* gene; and a deletion of *mqo* gene, *poxB* gene and/or *sdhABCD* genes, and further comprising a deletion of *glcDEF* genes, *lhgO* gene and/or *putA* gene, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene; a deletion of *mqo* gene, *poxB* gene and/or *sdhABCD* genes, and further comprising a deletion of *glcDEF* genes, *lhgO* gene and/or *putA* gene, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene; a deletion of *mqo* gene, and further comprising a deletion of *IIdD* gene and/or *dadA* gene.

Even more preferably, the present invention relates to a genetically engineered bacteria comprising a deletion of *ndh* gene, a deletion of *nuoEFG* genes, a deletion of *dld* gene; a deletion of *mqo* gene, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene; a deletion of *poxB* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene; a deletion of *poxB* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *putA* gene, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *putA* gene, and further comprising a deletion of *lldD* gene and/or *dadA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, and further comprising a deletion of *IIdD* gene and/or *dadA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, and further comprising a deletion of *IIdD* gene and/or *dadA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, and further comprising a deletion of *lldD* gene and/or *dadA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, and further comprising a deletion of *lldD* gene and/or *dadA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, and further comprising a deletion of *lldD* gene and/or *dadA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *lhgO* gene, and further comprising a deletion of *lldD* gene and/or *dadA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *putA* gene, and further comprising a deletion of *lldD* gene and/or *dadA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *putA* gene, and further comprising a deletion of *IIdD* gene and/or *dadA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, a deletion of *putA* gene, and further comprising a deletion of *lldD* gene and/or *dadA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, a deletion of *putA* gene, and further comprising a deletion of *lldD* gene and/or *dadA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *lldD* gene and/or *dadA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *lhgO* gene, a deletion of *putA* gene, and further comprising a deletion of *lldD* gene and/or *dadA* gene.

In preferred embodiments, the genetically engineered bacteria may further comprise a deletion of *kefF* gene and/or *mdaB-ygiN* genes.

Preferably, the present invention preferably relates to a genetically engineered bacteria comprising a deletion of *ndh* gene, a deletion of one or more *nuo* genes, a deletion of *dld* gene; and further comprising a deletion of *kefF* gene and/or *mdaB-ygiN* genes.

More preferably, the present invention preferably relates to a genetically engineered bacteria comprising a deletion of *ndh* gene, a deletion of *nuoEFG* genes, a deletion of *dld* gene; and further comprising a deletion of *kefF* gene and/or *mdaB-ygiN* genes.

Preferably, the present invention preferably relates to a genetically engineered bacteria comprising a deletion of *ndh* gene, a deletion of one or more *nuo* genes, a deletion of *dld* gene; and further comprising a deletion of *kefF* gene.

More preferably, the present invention preferably relates to a genetically engineered bacteria comprising a deletion of *ndh* gene, a deletion of *nuoEFG* genes, a deletion of *dld* gene; and further comprising a deletion of *kefF* gene.

Preferably, the present invention preferably relates to a genetically engineered bacteria comprising a deletion of *ndh* gene, a deletion of one or more *nuo* genes, a deletion of *dld* gene; and further comprising a deletion of *mdaB-ygiN* genes.

More preferably, the present invention preferably relates to a genetically engineered bacteria comprising a deletion of *ndh* gene, a deletion of *nuoEFG* genes, a deletion of *dld* gene; and further comprising a deletion of *mda8-ygiN* genes.

Preferably, the present invention preferably relates to a genetically engineered bacteria comprising a deletion of *ndh* gene, a deletion of one or more *nuo* genes, a deletion of *dld* gene; and further comprising a deletion of *kefF* gene and *mdaB -ygiN* genes.

More preferably, the present invention preferably relates to a genetically engineered bacteria comprising a deletion of *ndh* gene, a deletion of *nuoEFG* genes, a deletion of *dld* gene; and further comprising a deletion of *kefF* gene and *mda8-ygiN* genes.

Even more preferably, the present invention relates to a genetically engineered bacteria comprising a deletion of *ndh* gene, a deletion of one or more *nuo* genes, a deletion of *dld* gene; a deletion of *mqo* gene, and further comprising a deletion of *kefF* gene and/or if *mdaB-ygiN* genes.

Even more preferably, the present invention relates to a genetically engineered bacteria comprising a deletion of *ndh* gene, a deletion of *nuoEFG* genes, a deletion of *dld* gene; a deletion of *mqo* gene, and further comprising a deletion of *kefF* gene and/or of *mdaB-ygiN* genes.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *kefF* gene and/or of *mdaB-ygiN* genes.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *kefF* gene and/or of *mdaB-ygiN* genes.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *kefF* gene and/or of *mdaB-ygiN* genes.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *kefF* gene and/or of *mda8-ygiN* genes.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, and further comprising a deletion of *kefF* gene and/or of *mdaB-ygiN* genes.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *kefF* gene and/or of *mda8-ygiN* genes.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *kefF* gene and/or of *mdaB-ygiN* genes.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *kefF* gene and/or of *mda8-ygiN* genes.

Even more preferably, the present invention relates to a genetically engineered bacteria comprising a deletion of *ndh* gene, a deletion of one or more *nuo* genes, a deletion of *dld* gene; a deletion of *poxB* gene, a deletion of *sdhABCD* genes, and further comprising a deletion of *kefF* gene and/or of *mdaB-ygiN* genes.

Even more preferably, the present invention relates to a genetically engineered bacteria comprising a deletion of *ndh* gene, a deletion of *nuoEFG* genes, a deletion of *dld* gene; a deletion of *poxB* gene, a deletion of *sdhABCD* genes, and further comprising a deletion of *kefF* gene and/or of *mda8-ygiN* genes.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *kefF* gene and/or of *mdaB-ygiN* genes.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *kefF* gene and/or of *mda8-ygiN* genes.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, and further comprising a deletion of *kefF* gene and/or of *mdaB-ygiN* genes.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, and further comprising a deletion of *kefF* gene and/or of *mdaB- ygiN* genes.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, and further comprising a deletion of *kefF* gene and/or of *mdaB-ygiN* genes.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *IhgO* gene, and further comprising a deletion of *kefF* gene and/or of *mdaB-ygiN* genes.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, and further comprising a deletion of *kefF* gene and/or *mdaB-ygiN* genes.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *lhgO* gene, and further comprising a deletion of *kefF* gene and/or of *mda8-ygiN* genes.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *putA* gene, and further comprising a deletion of *kefF* gene and/or of *mda8-ygiN* genes.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *putA* gene, and further comprising a deletion of *kefF* gene and/or of *mdaB-ygiN* genes.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, a deletion of *putA* gene, and further comprising a deletion of *kefF* gene and/or of *mdaB-ygiN* genes.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, a deletion of *putA* gene, and further comprising a deletion of *kefF* gene and/or of *mdaB-ygiN* genes.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *kefF* gene and/or of *mdaB-ygiN* genes.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *glcDEF* genes, a deletion of *IhgO* gene, a deletion of *putA* gene, and further comprising a deletion of *kefF* gene and/or of *mdaB -ygiN* genes.

Even more preferably, the present invention relates to a genetically engineered bacteria comprising a deletion of *ndh* gene, a deletion of one or more *nuo* genes, a deletion of *dld* gene; a deletion of *mqo* gene, and further comprising a deletion of *IIdD* gene and/or *dadA* gene, and further comprising a deletion of *kefF* gene and/or of *mdaB-ygiN* genes.

Even more preferably, the present invention relates to a genetically engineered bacteria comprising a deletion of *ndh* gene, a deletion of *nuoEFG* genes, a deletion of *dld* gene; a deletion of *mqo* gene, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *lldD* gene and/or *dadA* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *lldD* gene and/or *dadA* gene, and further comprising a deletion of *kefF* gene and/or of *mdaB-ygiN* genes.

Even more preferably, the present invention relates to a genetically engineered bacteria comprising a deletion of *ndh* gene, a deletion of one or more *nuo* genes, a deletion of *dld* gene; a deletion of *poxB* gene, a deletion of *sdhABCD* genes, and further comprising a deletion of *lldD* gene and/or *dadA* gene, and further comprising a deletion of *kefF* gene and/or of *mdaB-ygiN* genes.

Even more preferably, the present invention relates to a genetically engineered bacteria comprising a deletion of *ndh* gene, a deletion of *nuoEFG* genes, a deletion of *dld* gene; a deletion of *poxB* gene, a deletion of *sdhABCD* genes, and further comprising a deletion of *lldD* gene and/or *dadA* gene, and further comprising a deletion of *kefF* gene and/or of *mdaB-ygiN* genes.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *lldD* gene and/or *dadA* gene, and further comprising a deletion of *kefF* gene and/or of *mdaB -ygiN* genes.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *lldD* gene and/or *dadA* gene, and further comprising a deletion of *kefF* gene and/or of *mdaB-ygiN* genes.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *putA* gene, and further comprising a deletion of *lldD* gene and/or *dadA* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *putA* gene, and further comprising a deletion of *IIdD* gene and/or *dadA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, and further comprising a deletion of *IIdD* gene and/or *dadA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, and further comprising a deletion of *IIdD* gene and/or *dadA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *IhgO* gene, and further comprising a deletion of *IIdD* gene and/or *dadA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, and further comprising a deletion of *lldD* gene and/or *dadA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, and further comprising a deletion of *lldD* gene and/or *dadA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *lhgO* gene, and further comprising a deletion of *lldD* gene and/or *dadA* gene, and further comprising a deletion of *kefF* gene and/or of *mdaB gene and*/*or of ygiN* genes.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *putA* gene, and further comprising a deletion of *lldD* gene and/or *dadA* gene and further comprising a deletion of *kefF* gene and/or of *mdaB gene and*/*or of ygiN* genes.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *putA* gene, and further comprising a deletion of *lldD* gene and/or *dadA* gene and further comprising a deletion of *kefF* gene and/or of *mdaB gene and*/*or of ygiN* genes.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, a deletion of *putA* gene, and further comprising a deletion of *lldD* gene and/or *dadA* gene and further comprising a deletion of *kefF* gene and/or of *mdaB gene and*/*or of ygiN* genes.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, a deletion of *putA* gene, and further comprising a deletion of *lldD* gene and/or *dadA* gene and further comprising a deletion of *kefF* gene and/or of *mdaB gene and*/*or of ygiN* genes.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *lldD* gene and/or *dadA* gene and further comprising a deletion of *kefF* gene and/or of *mdaB gene and*/*or of ygiN* genes.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *lhgO* gene, a deletion of *putA* gene, and further comprising a deletion of *lldD* gene and/or *dadA* gene and further comprising a deletion of *kefF* gene and/or of *mdaB gene and*/*or of ygiN* genes.

In preferred embodiments, the genetically engineered bacteria may further comprise a deletion of *gIpABC* genes and/or of *gpsA* gene.

Preferably, the present invention preferably relates to a genetically engineered bacteria comprising a deletion of *ndh* gene, a deletion of one or more *nuo* genes, a deletion of *dld* gene; and further comprising a deletion of *glpABC* genes and/or *of gpsA* gene.

More preferably, the present invention preferably relates to a genetically engineered bacteria comprising a deletion of *ndh* gene, a deletion of *nuoEFG* genes, a deletion of *dld* gene; and further comprising a deletion of *glpABC* genes and/or of *gpsA* gene.

Preferably, the present invention preferably relates to a genetically engineered bacteria comprising a deletion of *ndh* gene, a deletion of one or more *nuo* genes, a deletion of *dld* gene; and further comprising a deletion of *glpABC* genes.

More preferably, the present invention preferably relates to a genetically engineered bacteria comprising a deletion of *ndh* gene, a deletion of *nuoEFG* genes, a deletion of *dld* gene; and further comprising a deletion of *glpABC* genes.

Preferably, the present invention preferably relates to a genetically engineered bacteria comprising a deletion of *ndh* gene, a deletion of one or more *nuo* genes, a deletion of *dld* gene; and further comprising a deletion of *gpsA* gene.

More preferably, the present invention preferably relates to a genetically engineered bacteria comprising a deletion of *ndh* gene, a deletion of *nuoEFG* genes, a deletion of *dld* gene; and further comprising a deletion of *gpsA* gene.

Preferably, the present invention preferably relates to a genetically engineered bacteria comprising a deletion of *ndh* gene, a deletion of one or more *nuo* genes, a deletion of *dld* gene; and further comprising a deletion of *glpABC* genes and *gpsA* gene.

More preferably, the present invention preferably relates to a genetically engineered bacteria comprising a deletion of *ndh* gene, a deletion of *nuoEFG* genes, a deletion of *dld* gene; and further comprising a deletion of *glpABC* genes and *gpsA* gene.

Even more preferably, the present invention relates to a genetically engineered bacteria comprising a deletion of *ndh* gene, a deletion of one or more *nuo* genes, a deletion of *dld* gene; a deletion of *mqo* gene, and further comprising a deletion of *gIpABC* genes and/or of *gpsA* gene.

Even more preferably, the present invention relates to a genetically engineered bacteria comprising a deletion of *ndh* gene, a deletion of *nuoEFG* genes, a deletion of *dld* gene; a deletion of *mqo* gene, and further comprising a deletion of *gIpABC* genes and/or *of gpsA* gene.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *glpABC* genes and/or *of gpsA* gene.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *glpABC* genes and/or of *gpsA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, and further comprising a deletion of *gIpABC* genes and/or *of gpsA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *gIpABC* genes and/or *of gpsA* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *glpABC* genes and/or *of gpsA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *glpABC* genes and/or of *gpsA* gene.

Even more preferably, the present invention relates to a genetically engineered bacteria comprising a deletion of *ndh* gene, a deletion of one or more *nuo* genes, a deletion of *dld* gene; a deletion of *poxB* gene, a deletion of *sdhABCD* genes, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene; a deletion of *poxB* gene, a deletion of *sdhABCD* genes, and further comprising a deletion of *gIpABC* genes and/or *of gpsA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *glpABC* genes and/or *of gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, and further comprising a deletion of *glpABC* genes and/or *of gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, and further comprising a deletion of *glpABC* genes and/or *of gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, and further comprising a deletion of *glpABC* genes and/or of *gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, and further comprising a deletion of *glpABC* genes and/or *of gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, and further comprising a deletion of *glpABC* genes and/or of *gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *lhgO* gene, and further comprising a deletion of *glpABC* genes and/or of *gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *putA* gene, and further comprising a deletion of *glpABC* genes and/or of *gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *putA* gene, and further comprising a deletion of *glpABC* genes and/or of *gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, a deletion of *putA* gene, and further comprising a deletion of *glpABC* genes and/or of *gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, a deletion of *putA* gene, and further comprising a deletion of *glpABC* genes and/or of *gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *glpABC* genes and/or *of gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *glcDEF* genes, a deletion of *lhgO* gene, a deletion of *putA* gene, and further comprising a deletion of *glpABC* genes and/or *of gpsA* gene.

Even more preferably, the present invention relates to a genetically engineered bacteria comprising a deletion of *ndh* gene, a deletion of one or more *nuo* genes, a deletion of *dld* gene; a deletion of *mqo* gene, and further comprising a deletion of *lldD* gene and/or *dadA* gene, and further comprising a deletion of *gIpABC* genes and/or of *gpsA* gene.

Even more preferably, the present invention relates to a genetically engineered bacteria comprising a deletion of *ndh* gene, a deletion of *nuoEFG* genes, a deletion of *dld* gene; a deletion of *mqo* gene, and further comprising a deletion of *lldD* gene and/or *dadA* gene, and further comprising a deletion of *glpABC* genes and/or of *gpsA* gene.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *lldD* gene and/or *dadA* gene, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *lldD* gene and/or *dadA* gene, and further comprising a deletion of *glpABC* genes and/or *of gpsA* gene.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *IIdD* gene and/or of *dadA* gene, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *lldD* gene and/or of *dadA* gene, and further comprising a deletion of *glpABC* genes and/or of *gpsA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, and further comprising a deletion of *lldD* gene and/or of *dadA* gene, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *lldD* gene and/or of *dadA* gene, and further comprising a deletion of *glpABC* genes and/or of *gpsA* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *lldD* gene and/or of *dadA* gene, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *lldD* gene and/or of *dadA* gene, and further comprising a deletion of *kefF* gene and/or of *mdaB and*/*or of ygiN* gene, and further comprising a deletion of *glpABC* genes and/or of *gpsA* gene.

Even more preferably, the present invention relates to a genetically engineered bacteria comprising a deletion of *ndh* gene, a deletion of one or more *nuo* genes, a deletion of *dld* gene; a deletion of *poxB* gene, a deletion of *sdhABCD* genes, and further comprising a deletion of *lldD* gene and/or of *dadA* gene, and further comprising a deletion of *kefF* gene and/or of *mdaB and*/*or ygiN* gene, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene; a deletion of *poxB* gene, a deletion of *sdhABCD* genes, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *lldD* gene and/or of *dadA* gene, and further comprising a deletion of *kefF* gene and/or of *mdaB gene and*/*or of ygiN* gene, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *putA* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *putA* gene, and further comprising a deletion of *lldD* gene and/or *dadA* gene, and further comprising a deletion of *kefF* gene and/or of *mdaB-ygiN* genes, and further comprising a deletion of *glpABC* genes and/or of *gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, and further comprising a deletion of *lldD* gene and/or of *dadA* gene, and further comprising a deletion of *kefF* gene and/or of *mdaB -ygiN* genes, and further comprising a deletion of *glpABC* genes and/or of *gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, and further comprising a deletion of *lldD* gene and/or *dadA* gene, and further comprising a deletion of *kefF* gene and/or of *mdaB -ygiN* genes, and further comprising a deletion of *glpABC* genes and/or of *gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, and further comprising a deletion of *lldD* gene and/or of *dadA* gene, and further comprising a deletion of *kefF* gene and/or of *mdaB- ygiN* genes, and further comprising a deletion of *glpABC* genes and/or *gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, and further comprising a deletion of *lldD* gene and/or of *dadA* gene, and further comprising a deletion of *kefF* gene and/or of *mdaB-ygiN* genes, and further comprising a deletion of *gIpABC* genes and/or of *gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, and further comprising a deletion of *lldD* gene and/or of *dadA* gene, and further comprising a deletion of *kefF* gene and/or of *mdaB-ygiN* genes, and further comprising a deletion of *glpABC* genes and/or *gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *lhgO* gene, and further comprising a deletion of *lldD* gene and/or *dadA* gene, and further comprising a deletion of *kefF* gene and/or of *mdaB* - *ygiN* genes, and further comprising a deletion of *glpABC* genes and/or of *gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or *gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion of *poxB* gene, a deletion of *sdhABCD* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene.

More preferably, the present invention relates to a genetically engineered bacteria comprising a deletion of *ndh* gene, a deletion of *nuoEFG* genes, a deletion of *dld* gene; a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene.

In preferred embodiments, the genetically engineered bacteria may further comprise a deletion of *wrbA* gene and/or of *yieF* gene.

Preferably, the present invention preferably relates to a genetically engineered bacteria comprising a deletion of *ndh* gene, a deletion of one or more *nuo* genes, a deletion of *dld* gene; and further comprising a deletion of *wrbA* gene and/or of *yieF* gene.

More preferably, the present invention preferably relates to a genetically engineered bacteria comprising a deletion of *ndh* gene, a deletion of *nuoEFG* genes, a deletion of *dld* gene; and further comprising a deletion of *wrbA* gene and/or of *yieF* gene.

Preferably, the present invention preferably relates to a genetically engineered bacteria comprising a deletion of *ndh* gene, a deletion of one or more *nuo* genes, a deletion of *dld* gene; and further comprising a deletion of *wrbA* gene.

More preferably, the present invention preferably relates to a genetically engineered bacteria comprising a deletion of *ndh* gene, a deletion of *nuoEFG* genes, a deletion of *dld* gene; and further comprising a deletion of *wrbA* gene.

Preferably, the present invention preferably relates to a genetically engineered bacteria comprising a deletion of *ndh* gene, a deletion of one or more *nuo* genes, a deletion of *dld* gene; and further comprising a deletion of *yieF* gene.

More preferably, the present invention preferably relates to a genetically engineered bacteria comprising a deletion of *ndh* gene, a deletion of *nuoEFG* genes, a deletion of *dld* gene; and further comprising a deletion of *yieF* gene.

Preferably, the present invention preferably relates to a genetically engineered bacteria comprising a deletion of *ndh* gene, a deletion of one or more *nuo* genes, a deletion of *dld* gene; and further comprising a deletion of *wrbA* gene and of *yieF* gene.

More preferably, the present invention preferably relates to a genetically engineered bacteria comprising a deletion of *ndh* gene, a deletion of *nuoEFG* genes, a deletion of *dId* gene; and further comprising a deletion of *wrbA* gene and of *yieF* gene.

Even more preferably, the present invention relates to a genetically engineered bacteria comprising a deletion of *ndh* gene, a deletion of one or more *nuo* genes, a deletion of *dld* gene; a deletion of *mqo* gene, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene; a deletion of *mqo* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *wrbA* gene and/or *yieF* gene.

Even more preferably, the present invention relates to a genetically engineered bacteria comprising a deletion of *ndh* gene, a deletion of one or more *nuo* genes, a deletion of *dld* gene; a deletion of *poxB* gene, a deletion of *sdhABCD* genes, and further comprising a deletion of *wrbA* gene and/or *yieF* gene.

Even more preferably, the present invention relates to a genetically engineered bacteria comprising a deletion of *ndh* gene, a deletion of *nuoEFG* genes, a deletion of *dld* gene; a deletion of *poxB* gene, a deletion of *sdhABCD* genes, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion of *poxB* gene, a deletion of *sdhABCD* genes, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion of *poxB* gene, a deletion of *sdhABCD* genes, a deletion of *glcDEF* genes, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *lhgO* gene, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *putA* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *glcDEF* genes, a deletion of *putA* gene, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, a deletion of *putA* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, a deletion of *putA* gene, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, and further comprising a deletion of *glcDEF* genes, a deletion of *lhgO* gene, a deletion of *putA* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene; a deletion of *mqo* gene, and further comprising a deletion of *lldD* gene and/or of *dadA* gene, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene; a deletion of *mqo* gene, and further comprising a deletion of *lldD* gene and/or of *dadA* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *lldD* gene and/or *dadA* gene, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *lldD* gene and/or of *dadA* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *lldD* gene and/or of *dadA* gene, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *lldD* gene and/or of *dadA* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, and further comprising a deletion of *lldD* gene and/or of *dadA* gene, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *lldD* gene and/or of *dadA* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *lldD* gene and/or of *dadA* gene, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *lldD* gene and/or of *dadA* gene, and further comprising a deletion of *kefF* gene and/or of *mdaB* gene and/or of *ygiN* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene; a deletion of *poxB* gene, a deletion of *sdhABCD* genes, and further comprising a deletion of *lldD* gene and/or of *dadA* gene, and further comprising a deletion of *kefF* gene and/or of *mdaB-ygiN* genes, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene; a deletion of *poxB* gene, a deletion of *sdhABCD* genes, and further comprising a deletion of *lldD* gene and/or of *dadA* gene, and further comprising a deletion of *kefF* gene and/or of *mdaB* gene and/or of *ygiN* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, and further comprising a deletion of *lldD* gene and/or of *dadA* gene, and further comprising a deletion of *kefF* gene and/or of *mdaB* gene and/or of *ygiN* gene, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion of *poxB* gene, a deletion of *sdhABCD* genes, and further comprising a deletion of *lldD* gene and/or of *dadA* gene, and further comprising a deletion of *kefF* gene and/or of *mdaB* gene and/or of *ygiN* gene, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *putA* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

Even more preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *putA* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion of *poxB* gene, a deletion of *sdhABCD* genes, a deletion of *glcDEF* genes, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *lhgO* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion *poxB* gene, a deletion of *sdhABCD* genes, a deletion of *lhgO* gene, and further comprising a deletion of *lldD* gene and/or of *dadA* gene, and further comprising a deletion of *kefF* gene and/or of *mdaB-ygiN* genes, and further comprising a deletion of *glpABC* genes and/or *gpsA* gene, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *lhgO* gene, and further comprising a deletion of *lldD* gene and/or of *dadA* gene, and further comprising a deletion of *kefF* gene and/or of *mdaB-ygiN* genes, and further comprising a deletion of *glpABC* genes and/or *gpsA* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion of *poxB* gene, a deletion of *sdhABCD* genes, a deletion of *glcDEF* genes, a deletion of *lhgO* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene; a deletion of *mqo* gene, a deletion of *poxB* gene, a deletion of *sdhABCD* genes, a deletion of *glcDEF* genes, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or *gpsA* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, a deletion of *glcDEF* genes, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene; a deletion of *mqo* gene, a deletion of *poxB* gene, a deletion of *sdhABCD* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

More preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene.

In preferred embodiments, the genetically engineered bacteria may further comprise a deletion of *fadE* gene.

In preferred embodiments, the genetically engineered bacteria may further comprise a deletion of *glpD* gene.

Even more preferably, the present invention relates to a genetically engineered bacteria comprising a deletion of *ndh* gene, a deletion of one or more *nuo* genes, a deletion of *dld* gene, a deletion of *mqo* gene, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *mqo* gene, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *poxB* gene, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *poxB* gene, a deletion of *sdhABCD* genes, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *poxB* gene, a deletion of *sdhABCD* genes, and further comprising a deletion of *wrbA* gene and/or of *yieF* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *glcDEF* genes, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion *poxB* gene, a deletion of *sdhABCD* genes, a deletion of *glcDEF* genes, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *lhgO* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, a deletion of *lhgO* gene, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *glcDEF* genes, a deletion of *lhgO* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion *poxB* gene, a deletion of *sdhABCD* genes, a deletion of *glcDEF* genes, a deletion of *lhgO* gene, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *glcDEF* genes, a deletion of *putA* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, a deletion of *glcDEF* genes, a deletion of *putA* gene, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *lhgO* gene, a deletion of *putA* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, a deletion of *lhgO* gene, a deletion of *putA* gene, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion *poxB* gene, a deletion of *sdhABCD* genes, and further comprising a deletion of *glcDEF* genes, a deletion of *lhgO* gene, a deletion of *putA* gene, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *mqo* gene, and further comprising a deletion of *lldD* gene and/or of *dadA* gene, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *mqo* gene, and further comprising a deletion of *lldD* gene and/or of *dadA* gene, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *poxB* gene, and further comprising a deletion of *lldD* gene and/or of *dadA* gene, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *poxB* gene, and further comprising a deletion of *lldD* gene and/or of *dadA* gene, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *sdhABCD* genes, and further comprising a deletion of *lldD* gene and/or of *dadA* gene, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *sdhABCD* genes, and further comprising a deletion of *lldD* gene and/or of *dadA* gene, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *poxB* gene, and further comprising a deletion of *lldD* gene and/or of *dadA* gene, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, and further comprising a deletion of *lldD* gene and/or of *dadA* gene, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *sdhABCD* genes, and further comprising a deletion of *lldD* gene and/or of *dadA* gene, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *sdhABCD* genes, and further comprising a deletion of *lldD* gene and/or of *dadA* gene, and further comprising a deletion of *kefF* gene and/or of *mdaB* gene and/or of *ygiN* gene, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *poxB* gene, a deletion of *sdhABCD* genes, and further comprising a deletion of *lldD* gene and/or of *dadA* gene, and further comprising a deletion of *kefF* gene and/or of *mdaB-ygiN* genes, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *poxB* gene, a deletion of *sdhABCD* genes, and further comprising a deletion of *lldD* gene and/or of *dadA* gene, and further comprising a deletion of *kefF* gene and/or of *mdaB-ygiN* genes, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, and further comprising a deletion of *lldD* gene and/or of *dadA* gene, and further comprising a deletion of *kefF* gene and/or of *mdaB-ygiN* genes, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, and further comprising a deletion of *lldD* gene and/or of *dadA* gene, and further comprising a deletion of *kefF* gene and/or of *mdaB-ygiN* genes, and further comprising a deletion of *fadE* gene.

Even more preferably, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *putA* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

Even more preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, a deletion of *putA* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *glcDEF* genes, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, a deletion of *glcDEF* genes, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *lhgO* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, a deletion of *lhgO* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *glcDEF* genes, a deletion of *lhgO* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, a deletion of *glcDEF* genes, a deletion of *lhgO* gene, and further comprising a deletion of *lldD* gene and/or of *dadA* gene, and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *glcDEF* genes, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion *poxB* gene, a deletion of *sdhABCD* genes, a deletion of *glcDEF* genes, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion *poxB* gene, a deletion of *sdhABCD* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or *gpsA* gene, and further comprising a deletion of *wrbA* gene and/or of *yieF* gene, and further comprising a deletion of *fadE* gene.

Moreover, the present invention relates to a genetically engineered bacteria comprising a deletion of *ndh* gene, a deletion of one or more *nuo* genes, a deletion of *dld* gene, a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

More preferably, the present invention relates to a genetically engineered bacteria comprising a deletion of *ndh* gene, a deletion of *nuoEFG* genes, a deletion of *dld* gene, a deletion of *mqo* gene, a deletion of *poxB* gene, a deletion of *sdhABCD* genes, a deletion of *glcDEF* genes, a deletion of *lhgO* gene, a deletion of *putA* gene, and further comprising a deletion of *lldD* gene and/or of *dadA* gene and further comprising a deletion of *kefF* gene and/or of *mdaB-ygiN* genes, and further comprising a deletion of *glpABC* genes and/or of *gpsA* gene, and further comprising a deletion of *wrbA* gene and/or of *yieF* gene, and further comprising a deletion of *fadE* gene.

A stable obligate fermenting bacteria was obtained by deletion of the *nuo* and *ndh* gene along with one or more of the genes *kefF, wrbA, yieF, mdaB, putA, glcDEF, dld, mqo, dadA, fadE, lldD, glpABC, lhgO poxB, sdhABCD* instead of the *ubiCA* genes.

This bacteria is still able to synthesize ubiquinone via the common ubiquinone biosynthesis pathway. The genes *kefF, wrbA, yieF, mdaB* encode enzymes with NADPH dehydrogenase activity, the genes *putA, glcDEF, dld, mqo, dadA, fadE, glpD, lldD, glpABC, lhgO* encode enzymes with reactions of NADH dehydrogenase bypassing activities and the genes *poxB, sdhABCD* encode enzymes with quinone reducing reactions from central metabolism.

The genetically engineered obligate fermenting bacteria according to the present invention preferably comprises a deletion of at least one of the nuo-genes selected from *nuoE, nuoF* or *nuoG* and the *ndh* gene and further comprises a deletion of one or more of the genes *kefF, wrbA, yieF, mdaB, putA, glcDEF, dld, mqo, dadA, fadE, glpD, lldD, glpABC, lhgO poxB, sdhABCD.*

In a preferred embodiment, the genetically engineered obligate fermenting bacteria according to the present invention preferably comprises a deletion of the genes *nuoEFG,* the *ndh* gene and further comprises a deletion of one or more of the genes *kefF, wrbA, yieF, mdaB, putA, glcDEF, dld, mqo, dadA, fadE, glpD*-*, lldD, glpABC, lhgO poxB, sdhABCD.*

In a further preferred embodiment, the genetically engineered obligate fermenting bacteria according to the present invention preferably comprises a deletion of at least one of the nuo-genes selected from *nuoE, nuoF* or *nuoG* and the *ndh* gene and further comprises a deletion of one or more of the genes *kefF, wrbA, yieF, mdaB, putA, glcDEF, dld, mqo, dadA, fadE, lldD, glpABC, lhgO poxB, sdhABCD,* but comprises *glpD,* so that it is able to metabolize glycerol to produce lactate.

In a preferred embodiment, the genetically engineered obligate fermenting bacteria according to the present invention preferably comprises a deletion of the genes *nuoEFG,* the *ndh* gene and further comprises a deletion of one or more of the genes *kefF, wrbA, yieF, mdaB, putA, glcDEF, dld, mqo, dadA, fadE, lldD, glpABC, lhgO poxB, sdhABCD,* but comprises *glpD,* so that it is able to metabolize glycerol to produce lactate.

In a preferred embodiment, the genetically engineered obligate fermenting bacteria according to the present invention preferably comprises a deletion of the *nuoEFG* genes, of the *ndh* gene and further comprises a deletion of the *kefF* gene.

In a preferred embodiment, the genetically engineered obligate fermenting bacteria according to the present invention preferably comprises a deletion of the *nuoEFG* genes, of the *ndh* gene and further comprises a deletion of the *wrbA* gene.

In a preferred embodiment, the genetically engineered obligate fermenting bacteria according to the present invention preferably comprises a deletion of the *nuoEFG* genes, of the *ndh* gene and further comprises a deletion of the *yieF* gene.

In a preferred embodiment, the genetically engineered obligate fermenting bacteria according to the present invention preferably comprises a deletion of the *nuoEFG* genes, of the *ndh* gene and further comprises a deletion of the *mdaB* gene.

In a preferred embodiment, the genetically engineered obligate fermenting bacteria according to the present invention preferably comprises a deletion of the *nuoEFG* genes, of the *ndh* gene and further comprises a deletion of the *putA* gene.

In a preferred embodiment, the genetically engineered obligate fermenting bacteria according to the present invention preferably comprises a deletion of the *nuoEFG* genes, of the *ndh* gene and of the *ubiCA* genes and further comprises a deletion of the *glcDEF* genes.

In a preferred embodiment, the genetically engineered obligate fermenting bacteria according to the present invention preferably comprises a deletion of the *nuoEFG* genes, of the *ndh* gene and further comprises a deletion of the *dadA* gene.

In a preferred embodiment, the genetically engineered obligate fermenting bacteria according to the present invention preferably comprises a deletion of the *nuoEFG* genes, of the *ndh* gene and further comprises a deletion of the *fadE* gene.

In a preferred embodiment, the genetically engineered obligate fermenting bacteria according to the present invention preferably comprises a deletion of the *nuoEFG* genes, of the *ndh* gene and further comprises a deletion of the *lldD* gene.

In a preferred embodiment, the genetically engineered obligate fermenting bacteria according to the present invention preferably comprises a deletion of the *nuoEFG* genes, of the *ndh* gene and further comprises a deletion of the *glpABC* genes.

In a preferred embodiment, the genetically engineered obligate fermenting bacteria according to the present invention preferably comprises a deletion of the *nuoEFG* genes, of the *ndh* gene and further comprises a deletion of the *lhgO* gene.

In a preferred embodiment, the genetically engineered obligate fermenting bacteria according to the present invention preferably comprises a deletion of the *nuoEFG* genes, of the *ndh* gene and further comprises a deletion of the *poxB* gene.

In a preferred embodiment, the genetically engineered obligate fermenting bacteria according to the present invention preferably comprises a deletion of the *nuoEFG* genes, of the *ndh* gene and further comprises a deletion of the *sdhABCD* genes.

In a preferred embodiment, the genetically engineered obligate fermenting bacteria according to the present invention preferably comprises a deletion of the *nuoEFG* genes, of the *ndh* gene and further comprises a deletion of the *dld* gene.

In a preferred embodiment, the genetically engineered obligate fermenting bacteria according to the present invention preferably comprises a deletion of the *nuoEFG* genes, of the *ndh* gene and further comprises a deletion of the *mqo* gene.

In a preferred embodiment, the genetically engineered obligate fermenting bacteria according to the present invention preferably comprises a deletion of the *nuoEFG* genes, of the *ndh* gene and further comprises a deletion of at least two, three or four genes selected from the genes *kefF, wrbA, yieF, mdaB, putA, glcDEF, dld, mqo, dadA, fadE, glpD*-*, lldD, glpABC, lhgO poxB,* and *sdhABCD.*

In a most preferred embodiment, the genetically engineered obligate fermenting bacteria according to the present invention preferably comprises a deletion of the *nuoEFG* genes, of the *ndh* gene and further comprises a deletion of the genes *kefF, wrbA, yieF, mdaB, putA, glcDEF, dld, mqo, dadA, fadE, glpD, lldD, glpABC, lhgO poxB,* and *sdhABCD.*

The term "**genetically engineered bacteria**", as used herein, relates to a bacteria, including a bacterial cell or a bacterial strain, that has been subjected to genetic manipulations, herein in particular subjected to gene deletions.

The term "**gene deletion**", or "**deletion mutation**" (symbol: delta, Δ), as used herein, relates in genetics to a mutation in which a part of a sequence of DNA is left out during DNA replication. Any number of nucleotides can be deleted.

In the context of the invention, deletion of a gene may be modification of a gene encoding a desired polypeptide to be produced by the cell and/or a gene encoding a polypeptide involved in production of a primary or secondary metabolite by the cell. The gene can be removed in its entirety, or as an alternative also the deletion of part of the gene might result in a reduction of the activity of the encoded protei

Deletion of a desired gene in the chromosome can be done with non-homologous as well as with homologous recombination. Homologous recombination is preferred, as it opens the opportunity to introduce, to remove or to simultaneously introduce and remove a functionality. With **homologous recombination** is intended, the transforming DNA further contains a DNA sequence that is homologous to a genomic target sequence of the specific cell to be engineered. The skilled person will understand that no 100% identity is required to obtain homologous recombination. A percentage identity of 80%, preferably 90%, more preferably 95%, 98% or 99% will also suffice. Generally, the DNA sequence of interest to be inserted in the chromosome by homologous recombination is flanked by homologous sequences with a sufficient length to enable homologous recombination.

For example, a DNA sequence encoding a selection marker, such as kanamicin resistence, is flanked by gene specific upstream and downstream sequences to allow deletion of a target genes and insertion at its place of the selection marker.

### Preferred Bacteria

The genetically engineered bacteria may preferably be *Enterobacteriaceae, Staphylococcaceae, Streptococcaceae, Lactobacillaceae, Vibrionaceae, Pasteurellaceae, Pseudomonidae, Bacilli, Corynebacteria, Gluconobacteria, Acetobacteria, Methylobacteria* and the like.

Since several bacterial genomes have been sequenced and are online available in different specific databases, as listed at en.wikipedia.org/wiki/List_of_sequenced_bacterial_genomes, a person of ordinary skill in the art can easily confirm homologous gene sets by sequence alignment, and in particular using amino acid sequences, which diverge less than gene sequences in the prokaryotes.

Therefore, a preferred embodiment of the present invention discloses a genetically engineered bacteria comprising the gene deletions disclosed above, wherein the genetically engineered bacteria is a facultativ anaerob bacteria selected from the group comprising *Enterobacteriaceae, Staphylococcaceae, Streptococcaceae, Lactobacillaceae, Vibrionaceae,* and *Pasteurellaceae.*

The genetic engineered bacteria with the desired gene deletions can be obtained by using a gene editing method known in the prior art, such as as classical homologous recombination using restriction enzymes and DNA ligase, lambda Red recombinases system, Rec recombinases system, ET recombination, flippase recombinase, and CRISPR/Cas method, as described in Current Protocol in Molecular Biology, Wiley.

The term or "***Escherichia coli***" (*E. coli*) as used herein, relates to the well-known Gram-negative, facultative anaerobic, rod-shaped, coliform bacterium of the genus *Escherichia.* The term "***Escherichia coli strain***"***,*** (*E. coli* strain) as used herein, relates to a subgroup within the species that has unique characteristics that distinguish it from other strains. The *E. coli* K-12 and B strains are well-known and used routinely in molecular biology as both a tool and a model organism. The *E*. *coli* strains K-12, B, C, and W are thought of as model organism strains. These are classified in Risk Group 1 in biosafety guidelines. The term "***Escherichia coli* K-12 strain**", (*E*. *coli* K-12 strain) as used herein, relates to an *E*. *coli* strain isolated from a stool sample of a patient convalescent from diphtheria and was labelled K-12 in 1922 at Stanford University. The "***Escherichia coli* K-12 strain MG1655**", Genotype: F- lambda- ilvG- rfb-50 rph-1 Serotype: OR:H48:K-was sequenced by the Blattner laboratory because it approximates wild-type *E*. *coli* and "has been maintained as a laboratory strain with minimal genetic manipulation, having only been cured of the temperate bacteriophage lambda and F plasmid by means of ultraviolet light and acridine orange, respectively." The mutations listed in the genotype are present in most *E*. *coli* K-12 strains and were probably acquired early in the history of the laboratory strain. An extensive list of *Escherichia coli* K-12 strain derivatives and their individual construction, genotypes, phenotypes, plasmids and phage information can be viewed at Ecoliwiki. *E*. *coli* MG1655 has been engineered to express the genes encoding an arabinose inducible lambda Red recombineering system and a rhamnose inducible flippase recombinase to allow fast turnover for multiple deletions. However, the present invention is not restricted to a particular *E.coli* strain and different *E*. *coli* strains are suitable for carry out the invention. For example,

Preferred *E*. *coli* strains within the scope of the present invention are selected from the group comprising: *E. coli* MG1655, BW25113 strain, W3110 strain, JM109 strain, B21 strain, BL21(D3) strain, HMS174 strain, RV308(DE3) strain, and AD494 strain.

Thus, the present invention relates to a genetically engineered *Escherichia coli* as disclosed above, wherein the *Escherichia coli* is an *E*. *coli* MG1655 or an *E*. *coli* MG1655 further comprising one or more recombination systems. Preferred recombination systems for the present invention are lambda Red recombinases, Rec recombinases, ET recombination, flippase recombinase.

The recombinase system of lambda Red recombinases comprises three phage-derived lambda Red proteins: Gam, Exo and Beta, which are necessary to complete dsDNA recombination (Murphy, K.C. Use of bacteriophage lambda recombination functions to promote gene replacement in Escherichia coli. J Bacteriol 180, 2063-2071 (1998*)*); Gam prevents degradation of foreign linear double stranded DNA by the E.coli nucleases, Exo degrades dsDNA to form a single stranded DNA (ssDNA) and Beta binding facilitates recombination. The exact mechanism on how a desired construct recombines with the chromosome in the presence of the three lambda Red proteins has been highly debated.

However the genetically engineered *E*. *coli* of the present invention can be obtained also by other gene editing methods, which are known to the skilled person of the art, such as classical homologous recombination using restriction enzymes and DNA ligase, CRISPR/Cas (Robb, G B 2019. Genome editing with CRISPR-Cas: an overview. Current Protocols Essential Laboratory Techniques, 19, e36).

Thus, the present invention also relates to a genetically engineered *E*. *coli* comprising a deletion of *ndh* gene, a deletion of one or more *nuo* genes, a deletion of *dld* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN.*

In an alternative embodiment, the genetically engineered *E*. *coli* comprises a deletion of *ndh* gene, a deletion of one or more *nuo* genes, a deletion of *dld* gene, a deletion of *mqo* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN.*

In a further alternative embodiment, the genetically engineered *E*. *coli* comprises a deletion of *ndh* gene, a deletion of one or more *nuo* genes, a deletion of *dld* gene, a deletion of *mqo* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN.*

In another alternative embodiment, the genetically engineered *E*. *coli* comprises a deletion of *ndh* gene, a deletion of one or more *nuo* genes, a deletion of *dld* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN.*

In a particular embodiment, the genetically engineered E. *coli* comprises a deletion of *ndh* gene, a deletion of one or more *nuo* genes, a deletion of *dld* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of *poxB* gene and/or a deletion of *sdhABCD* genes.

In a more particular embodiment, the genetically engineered *E*. *coli* comprises a deletion of *ndh* gene, a deletion of one or more *nuo* genes, a deletion of *dld* gene, a deletion of *mqo* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of *poxB* gene and/or a deletion of *sdhABCD* genes.

In a still more particular embodiment, the genetically engineered E. *coli* comprises a deletion of *ndh* gene, a deletion of one or more *nuo* genes, a deletion of *dld* gene, a deletion of *mqo* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of *poxB* gene and/or a deletion of *sdhABCD* genes.

In a further more particular embodiment, the genetically engineered *E*. *coli* comprises a deletion of *ndh* gene, a deletion of one or more *nuo* genes, a deletion of *dld* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of *poxB* gene and/or a deletion of *sdhABCD* genes.

In a further particular embodiment, the genetically engineered *E*. *coli* comprises a deletion of *ndh* gene, a deletion of one or more *nuo* genes, a deletion of *dld* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and a deletion of one or more genes selected from the group comprising *glcDEF* genes, *of lhgO* gene,of *putA* gene, of *lldD* gene and/or of *dadA* gene..

In a further more particular embodiment, the genetically engineered *E*. *coli* comprises a deletion of *ndh* gene, a deletion of one or more *nuo* genes, a deletion of *dld* gene, a deletion of *mqo* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and a deletion of one or more genes selected from the group comprising *glcDEF* genes, *of lhgO* gene,of *putA* gene, of *lldD* gene and/or of *dadA* gene.

In a still further more particular embodiment, the genetically engineered *E*. *coli* comprises a deletion of *ndh* gene, a deletion of one or more *nuo* genes, a deletion of *dld* gene, a deletion of *mqo* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and a deletion of one or more genes selected from the group comprising *glcDEF* genes, *of lhgO* gene,of *putA* gene, of *lldD* gene and/or of *dadA* gene.

In other embodiment, the genetically engineered *E*. *coli* comprises a deletion of *ndh* gene, a deletion of one or more *nuo* genes, a deletion of *dld* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and a deletion of one or more genes selected from the group comprising *glcDEF* genes, *of lhgO* gene,of *putA* gene, of *lldD* gene and/or of *dadA* gene..

In other preferred embodiment, the genetically engineered *E*. *coli* comprises a deletion of *ndh* gene, a deletion of one or more *nuo* genes, a deletion of *dld* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* further comprising a deletion of *poxB* gene and/or a deletion of *sdhABCD* genes, and a deletion of one or more genes selected from the group comprising *glcDEF* genes, *of IhgO* gene,of *putA* gene, of *lldD* gene and/or of *dadA* gene..

In other more preferred embodiment, the genetically engineered *E*. *coli* comprises a deletion of *ndh* gene, a deletion of one or more *nuo* genes, a deletion of *dld* gene, a deletion of *mqo* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* further comprising a deletion of *poxB* gene and/or a deletion of *sdhABCD* genes, and a deletion of one or more genes selected from the group comprising *glcDEF* genes, *of lhgO* gene,of *putA* gene, of *lldD* gene and/or of *dadA* gene..

In other still more preferred embodiment, the genetically engineered *E*. *coli* comprises a deletion of *ndh* gene, a deletion of one or more *nuo* genes, a deletion of *dld* gene, a deletion of *mqo* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* further comprising a deletion of *poxB* gene and/or a deletion of *sdhABCD* genes, and a deletion of one or more genes selected from the group comprising *glcDEF* genes, *of lhgO* gene,of *putA* gene, of *lldD* gene and/or of *dadA* gene.

In other further more preferred embodiment, the genetically engineered *E*. *coli* comprises a deletion of *ndh* gene, a deletion of one or more *nuo* genes, a deletion of *dld* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of *poxB* gene and/or a deletion of *sdhABCD* genes, and a deletion of one or more genes selected from the group comprising *glcDEF* genes, *of lhgO* gene,of *putA* gene, of *lldD* gene and/or of *dadA* gene.

In a still more preferred embodiment, the genetically engineered E. *coli* comprises a deletion of *ndh* gene, a deletion of one or more *nuo* genes, a deletion of *dld* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of one or more genes selected from the group comprising *kefF* gene, *mdaB-ygiN* genes, *gIpABC* genes, *gpsA* gene, *wrbA* gene, *yieF* gene, and *fadE* gene.

The present invention preferably refers to a genetically engineered *E*. *coli* comprising a deletion of *ndh* gene, a deletion of one or more *nuo* genes, a deletion of *dld* gene, a deletion of *mqo* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of one or more genes selected from the group comprising *kefF* gene, *mdaB-ygiN* genes, *gIpABC* genes, *gpsA* gene, *wrbA* gene, *yieF* gene, and *fadE* gene.

The present invention more preferably refers to a genetically engineered *E*. *coli* comprising a deletion of *ndh* gene, a deletion of one or more *nuo* genes, a deletion of *dld* gene, a deletion of *mqo* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

The present invention still more preferably refers to a genetically engineered *E*. *coli* comprising a deletion of *ndh* gene, a deletion of one or more *nuo* genes, a deletion of *dld* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

An embodiment of the invention is also directed to a genetically engineered *E*. *coli* comprising a deletion of *ndh* gene, a deletion of one or more *nuo* genes, a deletion of *dld* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of *poxB* gene and/or a deletion of *sdhABCD* genes, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment of the invention is directed to a genetically engineered *E*. *coli* comprising a deletion of *ndh* gene, a deletion of one or more *nuo* genes, a deletion of *dld* gene, a deletion of *mqo* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of *poxB* gene and/or a deletion of *sdhABCD* genes, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A more preferred embodiment of the invention is directed to a genetically engineered E. *coli* comprising a deletion of *ndh* gene, a deletion of one or more *nuo* genes, a deletion of *dld* gene, a deletion of *mqo* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of *poxB* gene and/or a deletion of *sdhABCD* genes, and further comprising a deletion of one or more genes selected from the group comprising *kefF* gene, *mdaB-ygiN* genes, *gIpABC* genes, *gpsA* gene, *wrbA* gene, *yieF* gene, and *fadE* gene.

A still more preferred embodiment of the invention is directed to a genetically engineered *E. coli* comprising a deletion of *ndh* gene, a deletion of one or more *nuo* genes, a deletion of *dld* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of *poxB* gene and/or a deletion of *sdhABCD* genes, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A further more preferred embodiment of the invention is directed to a genetically engineered *E*. *coli* comprising a deletion of *ndh* gene, a deletion of one or more *nuo* genes, a deletion of *dld* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A still preferred embodiment of the invention is directed to a genetically engineered *E*. *coli* comprising a deletion of *ndh* gene, a deletion of one or more *nuo* genes, a deletion of *dld* gene, a deletion of *mqo* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A further preferred embodiment of the invention is directed to a genetically engineered *E*. *coli* comprising a deletion of *ndh* gene, a deletion of one or more *nuo* genes, a deletion of *dld* gene, a deletion of *mqo* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A still more preferred embodiment of the invention is directed to a genetically engineered *E. coli* comprising a deletion of *ndh* gene, a deletion of one or more *nuo* genes, a deletion of *dld* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered E. *coli* comprising a deletion of *ndh* gene, a deletion of one or more *nuo* genes, a deletion of *dld* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* further comprising a deletion of *poxB* gene and/or a deletion of *sdhABCD* genes, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A more preferred embodiment is directed to a genetically engineered *E*. *coli* comprising a deletion of *ndh* gene, a deletion of one or more *nuo* genes, a deletion of *dld* gene, a deletion of *mqo* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* further comprising a deletion of *poxB* gene and/or a deletion of *sdhABCD* genes, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A further preferred embodiment is directed to a genetically engineered *E. coli* comprising a deletion of *ndh* gene, a deletion of one or more *nuo* genes, a deletion of *dld* gene, a deletion of *mqo* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* further comprising a deletion of *poxB* gene and/or a deletion of *sdhABCD* genes, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A more preferred embodiment is directed to a genetically engineered *E*. *coli* comprising a deletion of *ndh* gene, a deletion of one or more *nuo* genes, a deletion of *dld* gene, a deletion of *glpD* gene, wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN,* and further comprising a deletion of *poxB* gene and/or a deletion of *sdhABCD* genes, a deletion of one or more genes selected from the group comprising *glcDEF* genes, of *lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A still more preferred embodiment is directed to a genetically engineered *E*. *coli* comprising a deletion of *ndh* gene, a deletion of *nuoE,* a deletion of *nuoF,* a deletion of *nuoG,* and a deletion of *dld* gene.

A still further preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, and a deletion of *mqo* gene.

An alternative embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, and a deletion of *glpD* gene.

An alternative preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, and a deletion of *glpD* gene.

An alternative more preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, and further comprising a deletion of *poxB* gene and/or a deletion of *sdhABCD* genes.

An alternative still more preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, and further comprising a deletion of *poxB* gene and/or a deletion of *sdhABCD* genes.

An alternative further preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, a deletion of *glpD* gene, and further comprising a deletion of *poxB* gene and/or a deletion of *sdhABCD* genes.

A further embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *glpD* gene, and further comprising a deletion of *poxB* gene and/or a deletion of *sdhABCD* genes.

An additional embodiment is directed to a genetically engineered *E*. *coli* comprising a deletion of *ndh* gene, a deletion of *nuoE,* a deletion of *nuoF,* a deletion of *nuoG,* a deletion of *dld* gene, and a deletion of one or more genes selected from the group comprising *glcDEF* genes, *of lhgO* gene,of *putA* gene, of *lldD* gene and/or of *dadA* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, and a deletion of one or more genes selected from the group comprising *glcDEF* genes, *of lhgO* gene,of *putA* gene, of *lldD* gene and/or of *dadA* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, a deletion of *glpD* gene, and a deletion of one or more genes selected from the group comprising *glcDEF* genes, *of lhgO* gene,of *putA* gene, of *lldD* gene and/or of *dadA* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *glpD* gene, and a deletion of one or more genes selected from the group comprising *glcDEF* genes, *of lhgO* gene,of *putA* gene, of *lldD* gene and/or of *dadA* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, further comprising a deletion of *poxB* gene and/or a deletion of *sdhABCD* genes, and a deletion of one or more genes selected from the group comprising *glcDEF* genes, *of lhgO* gene,of *putA* gene, of *lldD* gene and/or of *dadA* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, further comprising a deletion of *poxB* gene and/or a deletion of *sdhABCD* genes, and a deletion of one or more genes selected from the group comprising *glcDEF* genes, *of lhgO* gene,of *putA* gene, of *lldD* gene and/or of *dadA* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, a deletion of *glpD* gene, further comprising a deletion of *poxB* gene and/or a deletion of *sdhABCD* genes, and a deletion of one or more genes selected from the group comprising *glcDEF* genes, *of lhgO* gene,of *putA* gene, of *lldD* gene and/or of *dadA* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *glpD* gene, and further comprising a deletion of *poxB* gene and/or a deletion of *sdhABCD* genes, and a deletion of one or more genes selected from the group comprising *glcDEF* genes, *of lhgO* gene,of *putA* gene, of *lldD* gene and/or of *dadA* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, a deletion of *glpD* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene a deletion of *nuoE,* a deletion of *nuoF,* a deletion of *nuoG,* a deletion of *dld* gene, a deletion of *glpD* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, further comprising a deletion of *poxB* gene and/or a deletion of *sdhABCD* genes, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, further comprising a deletion of *poxB* gene and/or a deletion of *sdhABCD* genes, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, a deletion of *glpD* gene, further comprising a deletion of *poxB* gene and/or a deletion of *sdhABCD* genes, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *glpD* gene, further comprising a deletion of *poxB* gene and/or a deletion of *sdhABCD* genes, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *gIpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, a deletion of *glpD* gene, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *glpD* gene, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *gIpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, further comprising a deletion of *poxB* gene and/or a deletion of *sdhABCD* genes, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered E. *coli* comprising a deletion of *ndh* gene, a deletion of *nuoE,* a deletion of *nuoF,* a deletion of *nuoG,* a deletion of *dld* gene, a deletion of *mqo* gene, further comprising a deletion of *poxB* gene and/or a deletion of *sdhABCD* genes, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

A preferred embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *mqo* gene, a deletion of *glpD* gene, further comprising a deletion of *poxB* gene and/or a deletion of *sdhABCD* genes, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

Another embodiment is directed to a genetically engineered *E*. *coli* comprising a deletion of *ndh* gene, a deletion of *nuoE,* a deletion of *nuoF,* a deletion of *nuoG,* a deletion of *dld* gene, a deletion of *glpD* gene, further comprising a deletion of *poxB* gene and/or a deletion of *sdhABCD* genes, a deletion of one or more genes selected from the group comprising *glcDEF* genes, *of lhgO* gene,of *putA* gene, of *lldD* gene and/or of *dadA* gene, and further comprising a deletion of one or more genes selected from the group comprising *kefF* gene, *mdaB-ygiN* genes, *glpABC* genes, *gpsA* gene, *wrbA* gene, *yieF* gene, and *fadE* gene.

Thus, the present invention preferably discloses a genetically engineered *E*. *coli* comprising a deletion of *ndh* gene, of *nuoEFG* genes, of *dld* gene, of *mqo* gene, *poxB* gene and *sdhABCD* genes; and further comprising a deletion of *glcDEF* genes, *of lhgO* gene, of *putA* gene, *of lldD* gene, *of dadA* gene, of *kefF* gene, *of mdaB-ygiN* gene, of *glpABC* genes, of *gpsA* gene, of *wrbA* gene, of *yieF* gene, *of fadE* gene, and optionally of *glpD.*

In alternatively preferred embodiments, the present invention related to a genetically engineered *E*. *coli* as described above, wherein the *E*. *coli* is an *E*. *coli* MG1655. In more preferred embodiments, the present invention related to a genetically engineered *E*. *coli* as described above, wherein the *E*. *coli* is an *E*. *coli* MG1655 further comprising one or more recombination systems selected from the group comprising lambda Red recombinases, Rec recombinases, and flippase recombinase.

### Method for Lactate Production

The present invention also relates to a method for producing D-lactate comprising the following steps:
a) providing a genetically engineered bacteria as described in the present invention above;
b) providing a culture medium comprising a carbon source;
c) culturing the genetically engineered bacteria under aerobic conditions in the culture medium of b) to produce a culture medium comprising D-lactate,
wherein the carbon source is selected from the group comprising glucose, fructose, xylose, arabinose, galactose, mannose, sucrose, cellobiose, hemicellulose, and glycerol or a combination thereof.

A particular embodiment of the present invention relates to a method for producing D-lactate comprising the following steps:
a) providing a genetically engineered bacteria comprising a deletion of *ndh* gene, of one or more *nuo* genes selected from *nuoA, nuoB, nuoC, nuoD, nuoE, nuoF, nuoG, nuoH, nuoI, nuoJ, nuoK, nuoL, nuoM* and *nuoN,* of *dld* gene, and optionally of *mqo* gene;
b) providing a culture medium comprising a carbon source;
c) culturing the genetically engineered bacteria under aerobic conditions in the culture medium of b) to produce a culture medium comprising D-lactate,
wherein the carbon source is selected from the group comprising glucose, fructose, xylose, arabinose, galactose, mannose, sucrose, cellobiose, hemicellulose, and glycerol or a combination thereof.

A more particular embodiment of the present invention relates to a method for producing D-lactate comprising the following steps:
a) providing a genetically engineered bacteria comprising a deletion of *ndh* gene, of one or more *nuo* genes selected from *nuoA, nuoB, nuoC, nuoD, nuoE, nuoF, nuoG, nuoH, nuoI, nuoJ, nuoK, nuoL, nuoM* and *nuoN,* of *dld* gene, optionally of *mqo* gene, and/or further comprising a deletion of one or more genes selected from the group comprising or consisting of *glpD gene, poxB* gene, *sdhABCD* genes, *glcDEF* genes, *lhgO* gene, *putA* gene, *lldD* gene, *dadA* gene, *kefF* gene, *mdaB-ygiN* genes, *glpABC* genes, *gpsA* gene, *wrbA* gene, *yieF* gene, *and fadE* gene;
b) providing a culture medium comprising a carbon source;
c) culturing the genetically engineered bacteria under aerobic conditions in the culture medium of b) to produce a culture medium comprising D-lactate,
wherein the carbon source is selected from the group comprising glucose, fructose, xylose, arabinose, galactose, mannose, sucrose, cellobiose, hemicellulose, and glycerol or a combination thereof.

An even more particular embodiment of the present invention relates to a method for producing D-lactate comprising the following steps:
a) providing a genetically engineered bacteria comprising a deletion of *ndh* gene, of one or more *nuo* genes selected from *nuoA, nuoB, nuoC, nuoD, nuoE, nuoF, nuoG, nuoH, nuoI, nuoJ, nuoK, nuoL, nuoM* and *nuoN,* of *dld* gene, of ***glpD* gene,** optionally of *mqo* gene, and/or further comprising a deletion of one or more genes selected from the group comprising or consisting of *poxB* gene, *sdhABCD* genes, *glcDEF* genes, *lhgO* gene, *putA* gene, *lldD* gene, *dadA* gene, *kefF* gene, *mdaB-ygiN* genes, *glpABC* genes, *gpsA* gene, *wrbA* gene, *yieF* gene, *and fadE* gene;
b) providing a culture medium comprising a carbon source;
c) culturing the genetically engineered bacteria under aerobic conditions in the culture medium of b) to produce a culture medium comprising D-lactate,
wherein the carbon source is selected from the group comprising glucose, fructose, xylose, arabinose, galactose, mannose, sucrose, cellobiose, and hemicellulose, or a combination thereof.

An even more particular embodiment of the present invention relates to a method for producing D-lactate comprising the following steps:
a) providing a genetically engineered bacteria comprising a deletion of *ndh* gene, of one or more *nuo* genes selected from *nuoA, nuoB, nuoC, nuoD, nuoE, nuoF, nuoG, nuoH, nuoI, nuoJ, nuoK, nuoL, nuoM* and *nuoN,* of *dld* gene, optionally of *mqo* gene, and/or further comprising a deletion of one or more genes selected from the group comprising or consisting of *poxB* gene, *sdhABCD* genes, *glcDEF* genes, *IhgO* gene, *putA* gene, *lldD* gene, *dadA* gene, *kefF* gene, *mdaB-ygiN* genes, *glpABC* genes, *gpsA* gene, *wrbA* gene, *yieF* gene, *and fadE* gene;
b) providing a culture medium comprising a glycerol;
c) culturing the genetically engineered bacteria under aerobic conditions in the culture medium of b) to produce a culture medium comprising D-lactate.

A particular embodiment of the present invention relates to a method for producing D-lactate comprising the following steps:
a) providing a genetically engineered *E*. *coli* strain comprising a deletion of *ndh* gene, of one or more *nuo* genes selected from *nuoA, nuoB, nuoC, nuoD, nuoE, nuoF, nuoG, nuoH, nuoI, nuoJ, nuoK, nuoL, nuoM* and *nuoN,* of *dld* gene, and optionally of *mqo* gene;
b) providing a culture medium comprising a carbon source;
c) culturing the genetically engineered *E*. *coli* strain under aerobic conditions in the culture medium of b) to produce a culture medium comprising D-lactate,
wherein the carbon source is selected from the group comprising glucose, fructose, xylose, arabinose, galactose, mannose, sucrose, cellobiose, hemicellulose, and glycerol or a combination thereof.

A more particular embodiment of the present invention relates to a method for producing D-lactate comprising the following steps:
a) providing a genetically engineered *E*. *coli* strain comprising a deletion of *ndh* gene, of one or more *nuo* genes selected from *nuoA, nuoB, nuoC, nuoD, nuoE, nuoF, nuoG, nuoH, nuoI, nuoJ, nuoK, nuoL, nuoM* and *nuoN,* of *dld* gene, optionally of *mqo* gene, and/or further comprising a deletion of one or more genes selected from the group comprising or consisting of *glpD gene, poxB* gene, *sdhABCD* genes, *glcDEF* genes, *lhgO* gene, *putA* gene, *lldD* gene, *dadA* gene, *kefF* gene, *mdaB-ygiN* genes, *glpABC* genes, *gpsA* gene, *wrbA* gene, *yieF* gene, *and fadE* gene;
b) providing a culture medium comprising a carbon source;
c) culturing the genetically engineered *E*. *coli* strain under aerobic conditions in the culture medium of b) to produce a culture medium comprising D-lactate,
wherein the carbon source is selected from the group comprising glucose, fructose, xylose, arabinose, galactose, mannose, sucrose, cellobiose, hemicellulose, and glycerol or a combination thereof.

An even more particular embodiment of the present invention relates to a method for producing D-lactate comprising the following steps:
a) providing a genetically engineered *E*. *coli* strain comprising a deletion of *ndh* gene, of one or more *nuo* genes selected from *nuoA, nuoB, nuoC, nuoD, nuoE, nuoF, nuoG, nuoH, nuoI, nuoJ, nuoK, nuoL, nuoM* and *nuoN,* of *dld* gene, of ***glpD* gene,** optionally of *mqo* gene, and/or further comprising a deletion of one or more genes selected from the group comprising or consisting of *poxB* gene, *sdhABCD* genes, *glcDEF* genes, *lhgO* gene, *putA* gene, *lldD* gene, *dadA* gene, *kefF* gene, *mdaB-ygiN* genes, *glpABC* genes, *gpsA* gene, *wrbA* gene, *yieF* gene, *and fadE* gene;
b) providing a culture medium comprising a carbon source;
c) culturing the genetically engineered *E*. *coli* strain under aerobic conditions in the culture medium of b) to produce a culture medium comprising D-lactate,
wherein the carbon source is selected from the group comprising glucose, fructose, xylose, arabinose, galactose, mannose, sucrose, cellobiose, and hemicellulose, or a combination thereof.

An even more particular embodiment of the present invention relates to a method for producing D-lactate comprising the following steps:
a) providing a genetically engineered *E*. *coli* strain comprising a deletion of *ndh* gene, of one or more *nuo* genes selected from *nuoA, nuoB, nuoC, nuoD, nuoE, nuoF, nuoG, nuoH, nuoI, nuoJ, nuoK, nuoL, nuoM* and *nuoN,* of *dld* gene, optionally of *mqo* gene, and/or further comprising a deletion of one or more genes selected from the group comprising or consisting of *poxB* gene, *sdhABCD* genes, *glcDEF* genes, *lhgO* gene, *putA* gene, *lldD* gene, *dadA* gene, *kefF* gene, *mdaB-ygiN* genes, *gIpABC* genes, *gpsA* gene, *wrbA* gene, *yieF* gene, *and fadE* gene;
b) providing a culture medium comprising a glycerol;
c) culturing the genetically engineered *E*. *coli* strain under aerobic conditions in the culture medium of b) to produce a culture medium comprising D-lactate.

In preferred embodiments of the method described above, the *E*. *coli* is an *E*. *coli* MG1655 or an *E*. *coli* MG1655 further comprising one or more recombination systems selected from the group comprising lambda Red recombinases, Rec recombinases, and flippase recombinase

In preferred embodiments, the present method can further comprise the following steps:
d) collecting the culture medium comprising D-lactate, and
e) isolating D-lactate from the culture medium.

### Description of the figures:

- **Figure 1**: shows lactate production from glucose by the NNMini strain. Measurements represent means and errors from three independent experiments.
- **Figure 2**: shows lactate production from glycerol by the NNMini +*glpD* strain. Measurements represent means and errors from three independent experiments.
- **Figure 3**: shows genes deleted in the NNMini strain and illustrates their respective functions. E.g. NAD(P)H-dehydrogenases and quinone dependent dehydrogenases involved in either mini-cycles or central metabolism in the NNMini strain.
- **Figure 4**: shows comparison of aerobic lactate production from glucose using the E. *coli* strains *ΔubiCA,* NNQ, and NNMini. The data shown represents means of three independent measurements. The strains were incubated in M9 minimal medium under ambient (aerobic) conditions in the presence of 20, 10, 5, 2.5, 1.25, 0.6, 0.3, 0.15 mM glucose (NNQ and NNMini) or in presence of 20, 11, 6.9, 4.7 mM glucose (Δ*ubiCA*)*.* After the cultures reached stationary growth phase, supernatant samples were taken and analysed by ion-chromatography.

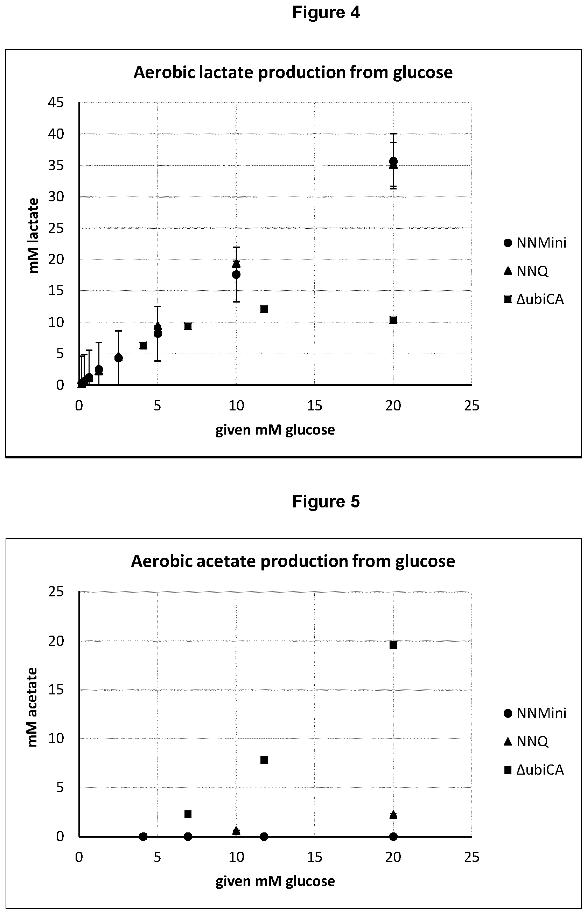

- **Figure 5**: shows comparison of aerobic acetate production from glucose using the following E. *coli* strains: (i) deleted in ubiquinone biosynthesis (*ΔubiCA*), (ii) deleted in both NADH dehydrogenases and ubiquinone biosynthesis (NNQ), and (iii) deleted in both NADH dehydrogenases as well as in reactions which might bypass NADH-dehydrogenase activity as described in XYZ and shown in Figure 5 (NNMini).The strains were incubated in M9 minimal medium under ambient (aerobic) conditions in the presence of 20, 10, 5, 2.5, 1.25, 0.6, 0.3, 0.15 mM glucose (NNQ and NNMini) or in presence of 20, 11, 6.9, 4.7 mM glucose (*ΔubiCA*)*.* After the cultures reached stationary growth phase, supernatant samples were taken and analysed by ion-chromatography. The data shown represents means of three independent measurements.
- **Figure 6**: shows comparison of aerobic lactate production from glycerol using the *E*. *coli* strains *ΔubiCA,* NNQ, and NNMini with reintroduced chromosomal glpD, i.e. NNMini glpD. The data represent means of three independent measurements.
- **Figure 7**: shows comparison of aerobic acetate production from glycerol using the following *E*. *coli* strains *ΔubiCA,* NNQ, and NNMini with reintroduced chromosomal glpD, i.e. NNMini glpD. The data represent means of three independent measurements.

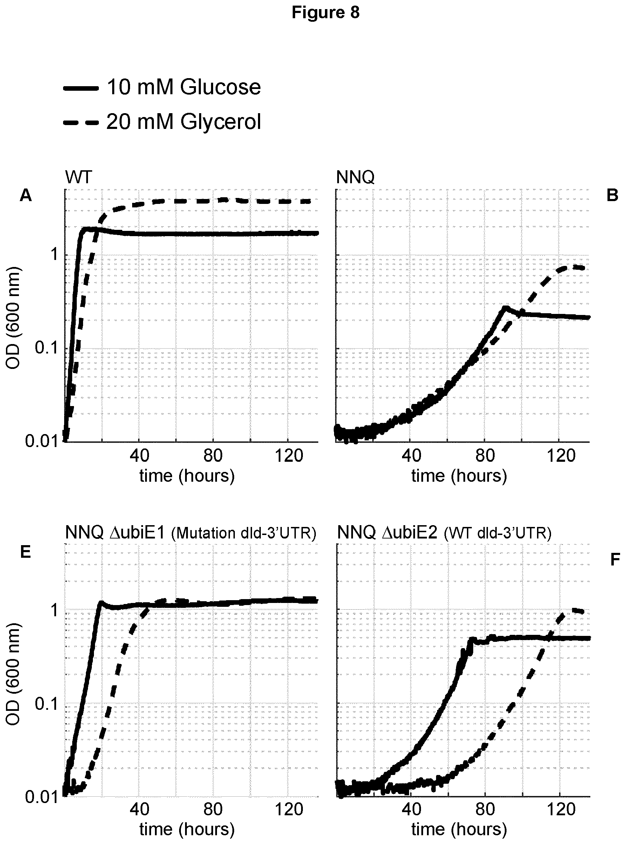

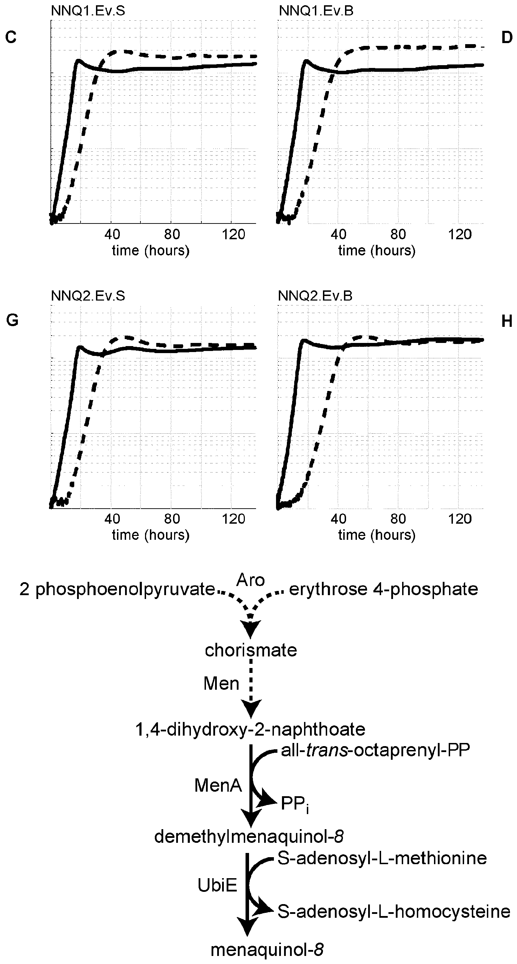

- **Figure 8**: shows growth a) on glucose or glycerol comprising minimal media of WT (A), NNQ (B), NNQ1 Ev.S (isolated evolved NNQ strain 1S) (C), NNQ1 Ev.B (isolated evolved NNQ strain 1B) (D), and of the reverse engineered strain NNQ *ΔubiE1* (mutation in did promotor) (E) and NNQ *ΔubiE2* (no mutation in did promotor) (F), mutants NNQ2 Ev.S (isolated evolved NNQ strain 1S) (G), NNQ2 Ev.B (isolated evolved NNQ strain 1B) (H). b) Mutations / Deletion of *ubiE* is known to result in accumulation of demethylmenaquinol.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Methods

**Strain and plasmids construction.** All *E*. *coli* strains used in this study are listed in Table 1. An *E*. *coli* MG1655 further expressing a λ-red recombinase system and a flippase recombinase, *E. coli* SIJ_488, was a gift from Alex Nielesen (Addgene palsmid #68246) and used as wildtype (E. *coli* WT). Gene deletions were performed by λ-Red recombineering or P1-transduction.

***E. coli* culture media.** LB medium (1% NaCl, 0.5% yeast extract, 1% tryptone) was used for cloning, generation of deletion strains, and strain maintenance. When appropriate, kanamycin (25 µg/mL), ampicillin (100 µg/mL), streptomycin (100 µg/mL), or chloramphenicol (30 µg/mL) were used. Growth experiments were carried out without antibiotics in standard M9 minimal medium free of any carbon source or amino acids, and containing 50 mM Na₂HPO₄, 20 mM KH₂PO₄, 1 mM NaCl, 20 mM NH₄Cl, 2 mM MgSO₄ and 100 µM CaCl₂, 134 µM EDTA, 13 µM FeCl₃·6H₂O, 6.2 µM ZnCl₂, 0.76 µM CuCl₂·2H₂O, 0.42 µM CoCl_{2·}2H₂O, 1.62 µM H₃BO₃, 0.081 µM MnCl₂·4H₂O. Carbon sources were added as indicated in the Examples and Figures.

**Analysis of supernatants with Ion Chromatography.** For detection of fermentation products, supernatants were analyzed by ion chromatography using the Dionex IonPac AS11-HC-4µm Analytical/Capillary Colum (Thermo Scientific, Dreieich, Germany) with suppressed conductivity detection. Standards of D-lactate, acetate and formate were prepared with deionized water. Supernatants from cell cultures were isolated by centrifugation for 10 minutes at 20,000*g and transfer of the supernatant to a new Eppendorf tube. Both standards and supernatants were diluted 100-fold with water. The analytes in a 10µl injection were separated using electrolytically generated potassium hydroxide eluent from 1 to 60mM KOH at 0.38ml/min within 53min at 10°C.

**Analysis of supernatants with High-Performance Anion-Exchange Chromatography.** For detection of alcohols and sugars, supernatants were analyzed by high-performance anion exchange chromatography using the Dionex CarboPac MA1 anion-exchange column (Thermo Scientific, Dreieich, Germany) with pulsed amperometric detection. Standards of D-glucose, D-xylose, glycerol and ethanol were prepared with deionized water. Supernatants from cell cultures were isolated by centrifugation for 10 minutes at 20.000*g and transfer of the supernatant to a new Eppendorf tube. The analytes in a 10 µl injection were separated using 480 mM sodium hydroxide eluent flowing at 0.4 ml/min for 60 min.

### Example 1 Gene deletion via P1 Phage transduction.

Gene deletions of *putA, dld, mqo, dadA, poxB, kefF, wrbA, fadE, yieF, glpD, lldO, lhgO* were generated by P1 phage transduction (Thomason et al., Curr Protoc Mol Biol. 2007, Ch. 1:1.17.1-1.17.8). Donor strains were strains JWxxxx from the KEIO collection listed in Table 1 with a kanamycin-resistance gene (KmR). The strains of E. coli with the desired deletions were obtained by genetic recombination with the P1 phage lysate and selected for by plating on kanamycin containing plates. A successful gene deletion was verified by determining the size of the genomic locus by PCR with DreamTaq polymerase (Thermo Scientific, Dreieich, Germany) and the respective KO-Ver primers (**Table 2**). Furthermore, a PCR with DreamTaq polymerase (Thermo Scientific, Dreieich, Germany) and internal primers ("int") binding inside of the gene coding sequence was performed to confirm that no copy of the gene to be deleted was present in the genome of the transduced strain. For removal of the selective marker, a fresh culture was grown to OD₆₀₀ ~ 0.2, followed by addition of 50 mM L-Rhamnose and cultivating for ~4h at 30°C for flippase expression induction. Colonies that only grew on LB medium in absence of the respective antibiotic were isolated and successful removal of the KmR gene from the respective locus was confirmed by PCR using the locus specific KO-Ver primers and with DreamTaq polymerase (Thermo Scientific, Dreieich, Germany).

### Example 2: Gene deletion by recombineering.

Gene deletion by recombineering involved PCR with "KO" primers (**Table 2**) with 50bp homologous overhangs and pKD4 plasmid (Addgene # 45605; http://n2t.net/addgene:45605; RRID: Addgene_45605) as template and PrimeStar GXL polymerase (Takara Bio) was performed to generate kanamycin resistance cassettes. *E. coli* WT cells were prepared for gene deletion by inoculating fresh cultures in LB, followed by induction of the recombinase genes by addition of 15 mM _{L}-arabinose at OD ~0.4-0.5, followed by incubation for 45 min at 37°C. The cells were harvested (11,000 rpm, 30 sec, 2°C) and washed three times with ice cold 10 % glycerol. For electroporation, ~300 ng of Km cassette PCR-product was transformed (1 mm cuvette, 1.8 kV, 25 µF, 200 Ω). Gene deletions were confirmed by selection on kanamycin containing plates and via one PCR using 'KO-Ver' primers (Table 2) and one PCR using internal (int) primers (Table 2), both of these being done with DreamTaq polymerase (Thermo Scientific, Dreieich, Germany). The Km cassette was removed by adding 50 mM _{L}-rhamnose to an exponentially growing 2 ml LB culture at OD 0.5 for induction of flippase gene expression. After induction, cells were incubated for ≥ 3 h at 30°C. After screening colonies for kanamycin sensitivity, removal of antibiotic resistance cassette was confirmed by PCR using 'KO-Ver' primers and DreamTaq polymerase (Thermo Scientific, Dreieich, Germany).

### Example 3: Growth analysis of mutant strains NNMini, NNMinimal and NNQ

Overnight cultures of **NNMini, NNMinimal** and **NNQ** were incubated in 4 mL M9 medium containing 20 mM glucose supplemented with 10mM acetate. Cultures were harvested (6,000**g*, 3 min) and washed three times in M9 medium to remove residual carbon sources. For growth analysis the washed cells were inoculated to an OD₆₀₀ of 0.01 in 96-well microtiter plates (Nunclon Delta Surface, Thermo Scientific) at 37°C, each well contained 150 µL of culture and to avoid evaporation while allowing gas exchange 50 µL mineral oil (Sigma-Aldrich). Growth in technical triplicates was monitored at 37°C in a BioTek Epoch 2 Microtiterplate reader (BioTek, Bad Friedrichshall, Germany) by absorbance measurements (600 nm) of each well every ~10 minutes with intermittent orbital and linear shaking. As previously established empirically for the instruments, blank measurements were subtracted and OD₆₀₀ measurements were converted to cuvette OD₆₀₀ values by multiplying with a factor of 4.35. Growth curves represent averages of measurements of technical replicates and were plotted in MATLAB.

### Example 4: Lactate and acetate production by the E. coli engineered strain grown on glucose.

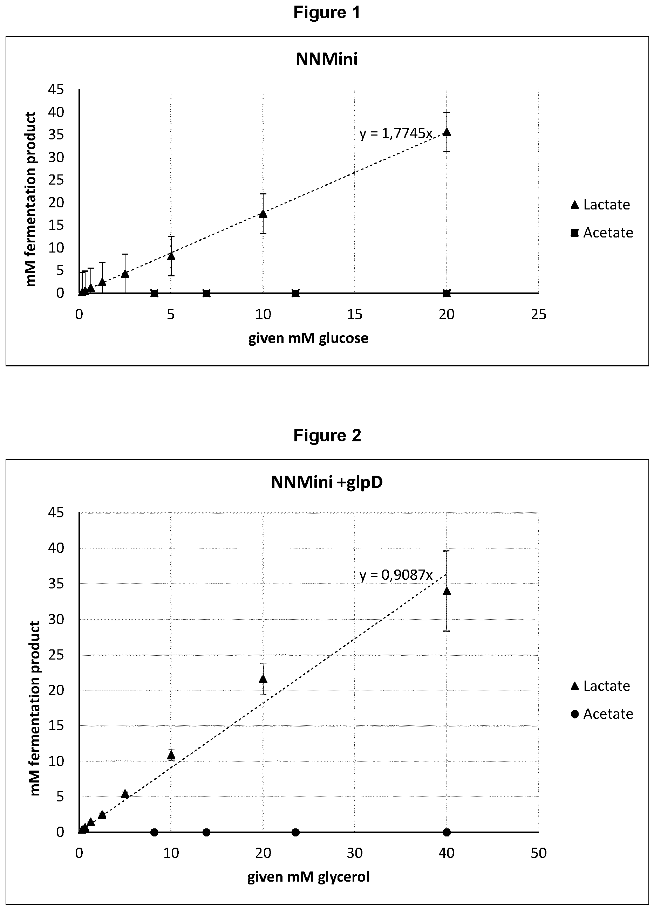

The NNMini strain *E*. *coli* was cultivated and grown in minimal medium in presence of increasing concentrations of glucose as explained in the method section. After the cultures reached stationary growth phase, the concentration of the fermentative products lactate and acetate were analyzed in the supernatants of the NNMini strain by Ion Chromatography. Results showed that in the NNMini strain supernatants, lactate concentrations were directly proportional to the **glucose** concentration in the medium, whereas no acetate could be detected (**Fig. 1**, *n*=3).

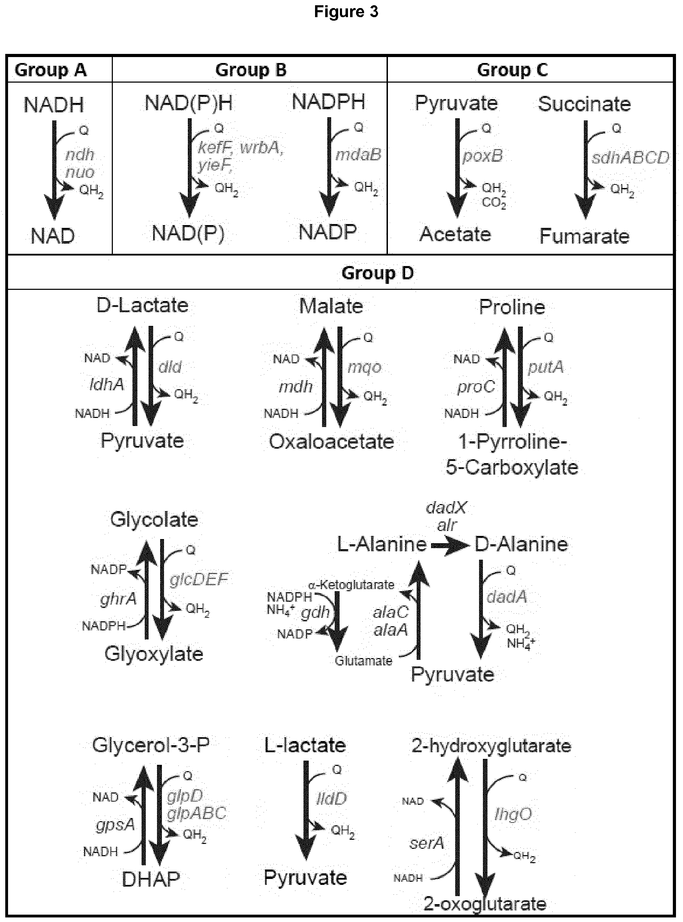

Thereafter, the production of lactose and acetate starting from glucose was analysed in the following engineered *E*. *coli* strains:
(i) *ΔubiCA,* deleted in ubiquinone biosynthesis,
(ii) *NNQ*, deleted in both NADH dehydrogenases and ubiquinone biosynthesis, and
(iii) *NNMini(glpD-),* deleted in both NADH dehydrogenases as well as in reactions which might bypass NADH-dehydrogenase activity, (**Fig. 3**), and also in *glpD* gene.

The strains were incubated in M9 minimal medium under ambient (aerobic) conditions in the presence of 20, 10, 5, 2.5, 1.25, 0.6, 0.3, 0.15 mM glucose (NNQ and NNMini) or in presence of 20, 11, 6.9, 4.7 mM glucose (*ΔubiCA*)*.* After the cultures reached stationary growth phase, supernatant samples were taken and analysed by ion-chromatography to determine lactose and acetate concentrations (**Fig. 4** and **5**, *n*=3).

The results (**Fig. 4**) showed that in the supernatants from NNMini(glpD-) and NNQ strains, lactate concentrations were directly proportional to the glucose concentration in the medium, whereas lactate production by *ΔubiCA* decreased at glucose concentrations higher than 10 mM.

Moreover, the results (**Fig. 5**) showed that in the supernatants from ***ΔubiCA*** strain, acetate concentrations were directly proportional to the glucose concentration in the medium. Some acetate production was detected also in the supernatants from NNQ strain, but at niveau much lower than in *ΔubiCA* supernatant, reaching values of 2.26 mM and 19.6 mM respectively at 20 mM glucose. In contrast, the *NNMini* strain did not produce any acetate.

The ability of acetate production under aerobic conditions indicates that NNQ and *ΔubiCA* strains are somehow able to transfer some electrons from pyruvate, either directly via PoxB to quinone (other than ubiquinone) or via NADH generated by pyruvate dehydrogenase followed by a leaky activity connecting NADH oxidation and quinone reduction (likely one of the activities / minicycles shown in Figure 3).

### Example 5: Lactate and acetate production by the E. coli engineered strain from glycerol.

The NNMini +glpD strain was cultivated and grown in minimal medium containing increasing glycerol concentrations 8.14, 13.8, 23.5, and 40 mM under ambient (aerobic) conditions (**Figure 2**). Results showed that in the NNMini+g/pD strain supernatants, lactate concentrations were directly proportional to the **glycerol** concentration in the medium, whereas no acetate could be detected (n=3).

Thereafter, the production of lactate and acetate starting from **glycerol** was analysed in the following engineered *E*. *coli* strains:
(i) *ΔubiCA,*
(ii) *NNQ,* and
(iii) *NNMini(glpD⁺)*, with reintroduced chromosomal glycerol 3-phosphate dehydrogenase (*glpD*)*.*

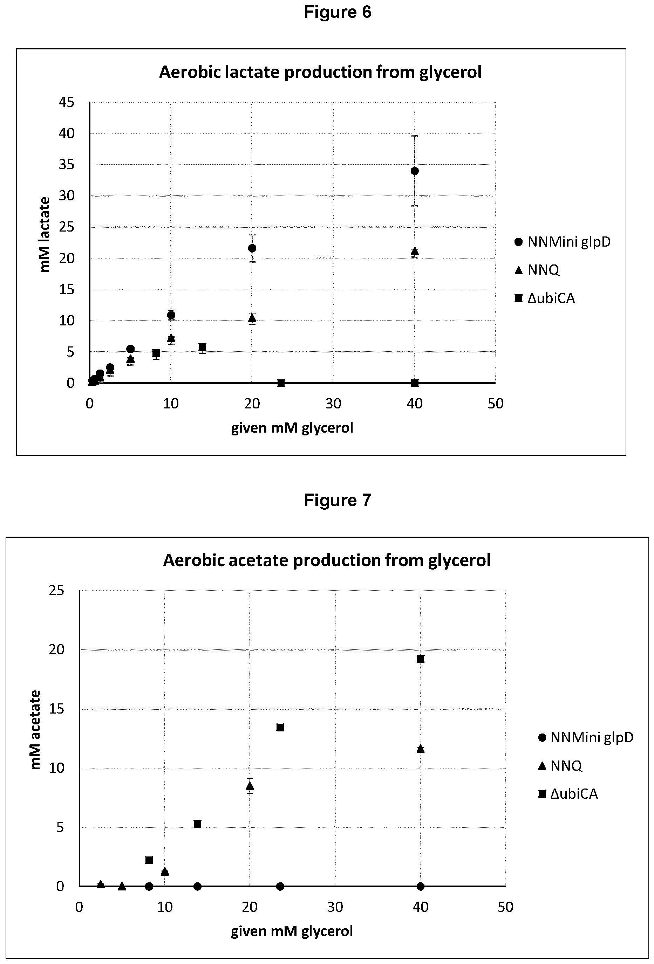

The strains were incubated in M9 minimal medium under ambient (aerobic) conditions in the presence of 40, 20, 10, 5, 2.5, 1.25, 0.6, 0.3 mM glycerol (NNMini glpD and NNQ) or 40, 23.5, 13.8, 8.1 mM glycerol (*ΔubiCA*)*.* After the cultures reached stationary growth phase, supernatant samples were taken and analysed by ion-chromatography to determine concentrations of lactate and acetate (**Fig. 6** and **7**, *n*=*3*)*.* The data shown represents means of three independent measurements.

The results indicated an improved lactate production curve by the NNMini glpD strain, compared to ΔubiCA and NNQ strains **(****Fig. 6****)**.

Moreover, ΔubiCA and NNQ strains from glycerol were able to produce acetate from glycerol (**Fig. 7****)**, while no acetate was produced by the NNMini glpD⁺ strain. The ability of acetate production under aerobic conditions indicates that NNQ and ΔubiCA strains are able to transfer some electrons from pyruvate, either via PoxB to quinone (other than ubiquinone) directly or via NADH generated by pyruvate dehydrogenase followed by a leaky activity connecting NADH oxidation and quinone reduction (likely one of the activities / minicycles shown in Figure 3).

### Example 6: Evolution of engineered strains

The results were performed to analyse the stability of the phenotypes of the enginnered strains. Evolved NNQ strains and the reverse engineered ubiE mutants in combination with did overexpression showed a wildtype like growth in several experiments on glucose or glycerol minimal media, indicating an activity of NADH dehydrogenase like mini-cycle between D-lactate and pyruvate.

Genome sequencing of isolates from these cultures revealed deleterious mutations in the ubiE gene together with a mutation in the promoter region of did (D-lactate dehydrogenase), which causes a transcription increase. Mutations / deletion of ubiE are known to result in accumulation of demethylmenaquinol.

Reverse engineering of this mutation by deleting the ubiE gene resulted in the loss of the fermentative phenotype, if the point mutation in the did promotor was introduced as well.

The graphs show growth of WT (A), NNQ (B), NNQ1 Ev.S (isolated evolved NNQ strain 1S) (C), NNQ1 Ev.B (isolated evolved NNQ strain 1B) (D). The reverse engineered strain NNQ ΔubiE1 (mutation in did promotor) (E) and NNQ ΔubiE2 (no mutation in did promotor) (F), mutants NNQ2 Ev.S (isolated evolved NNQ strain 1S) (G), NNQ2 Ev.B (isolated evolved NNQ strain 1B) (H).

Additionally, IC analysis revealed the absence of fermentative products. Thus, indicating the instability of the fermentative phenotype of the NNQ strain. While a deletion of did might restore the fermentative phenotype, similar events might lead to the activation of other minicycles (figure 3).

These results indicate that a simple mutation can circumvent the obligate fermentation phenotype in the NNQ strain.

In none of our experiments the NNMini strain ever circumvented the selection. Therefore, this characteristic is due to the combination of the additional deletions of the genes *poxB, sdhABCD, glcDEF, IhgO, putA, dld, lldD, dadA, kefF, mdaB, ygiN glpABC, gpsA, wrbA, yieF, and fadE.*

**Table 1: E. coli strains used in this study**

| **Name** | **Description and genotype** | **Source** |
|---|---|---|
| DH5α | Cloning of overexpression constructs | |
| *E. coli* SIJ_488 (WT) | *E. coli* K-12 MG1655 Tn7::para-exo-beta-gam; prha-FLP; xylSpm-lscel | Jensen et al. 2015. Sci Rep. 5:17874. |
| NNQ | Δ*ndh ΔnuoEFG* Δ*ubiCA* | This study |
| NNMini | Δ*ndh* Δ*nuoEFG* Δ*putA* Δ*glcDEF* Δ*dld* Δ*mqo* Δ*dadA* Δ*poxB ΔkefF ΔwrbA* Δ*fadE* Δ*yieF* Δ*mdaB-ygiN* Δ*glpD ΔlldD* Δ*glpABC ΔsdhABCD* Δ*lhgO* | This study |
| NNMini+*glpD* | Δ*ndh* Δ*nuoEFG* Δ*putA* Δ*glcDEF* Δ*dld* Δ*mqo* Δ*dadA* Δ*poxB* Δ*kefF* Δ*wrbA* Δ*fadE* Δ*yieF* Δ*mdaBygiN* Δ*lldD* Δ*glpABC* Δ*sdhABCD ΔlhgO* | This study |
| NNMinimal1 | Δ*ndh* Δ*nuoEFG Δdld* | This study |
| NNMinimal2 | Δ*ndh* Δ*nuoEFG* Δ*dld* Δ*mqo* | This study |
| JW0999 | *ΔputA donor:* | Keio collection |
| | *F-*, *Δ(araD-araB)567, ΔlacZ4787(::rrnB-3), λ⁻, ΔputA758::kan, rph-1, Δ(rhaD-rhaB)568, hsdR514* | |
| JW2121 | *Δdld donor:* | Keio collection |
| | *F-, Δ(araD-araB)567, ΔlacZ4787(::rrnB-3), λ⁻, Δdld-759::kan, rph-1, Δ(rhaD-rhaB)568, hsdR514* | |
| JW2198 | Δ*mqo donor:* | Keio collection |
| | *F-, Δ(araD-araB)567, ΔlacZ4787(::rrnB-3), λ-, Δmqo-763::kan, rph-1, Δ(rhaD-rhaB)568, hsdR514* | |
| JW1178 | Δ*dadA donor:* | Keio collection |
| | *F-, Δ(araD-araB)567, ΔlacZ4787(::rrnB-3), λ-, ΔdadA778::kan, rph-1, Δ(rhaD-rha8)568, hsdR514* | |
| JW0855 | Δ*poxB donor:* | Keio collection |
| | *F-, Δ(araD-araB)567, ΔlacZ4787(::rrnB-3), λ-, Δpox8772::kan, rph-1, Δ(rhaD-rhaB)568, hsdR514* | |
| JW0045 | *ΔkefF donor::* | Keio collection |
| | F-, ΔkefF759::kan, Δ(araD-araB)567, ΔlacZ4787(::rrnB-3), λ*-,* rph-1, Δ(rhaD-rhaB)568, hsdR514 | |
| JW0989 | Δ*wrbA donor::* | Keio collection |
| | F-, Δ(araD-araB)567, ΔlacZ4787(::rrnB-3), λ-, ΔwrbA748::kan, rph-1, Δ(rhaD-rhaB)568, hsdR514 | |
| JW5020 | Δ*fadE donor::* | Keio collection |
| | F-, Δ(araD-araB)567, ΔfadE739::kan, ΔlacZ4787(::rrnB-3), λ*-,* rph-1, Δ(rhaD-rhaB)568, hsdR514 | |
| JW3691 | Δ*yieF donor::* | Keio collection |
| | F-, Δ(araD-araB)567, ΔlacZ4787(::rrnB-3), λ*-,* rph-1, ΔyieF744::kan, Δ(rhaD-rhaB)568, hsdR514 | |
| JW3389 | Δ*glpD donor::* | Keio collection |
| | F-, Δ(araD-araB)567, ΔlacZ4787(::rrnB-3), λ-, ΔglpD759::kan, rph-1, Δ(rhaD-rhaB)568, hsdR514 | |
| JW3580 | Δ*lldD donor::* | Keio collection |
| | F-, Δ(araD-araB)567, ΔlacZ4787(::rrnB-3), λ-, ΔlldD789::kan, rph-1, Δ(rhaD-rhaB)568, hsdR514 | |
| JW2635 | Δ*lhgO donor::* | Keio collection |
| | F-, Δ(araD-araB)567, ΔlacZ4787(::rrnB-3), λ-, ΔygaF741::kan, rph-1, Δ(rhaD-rhaB)568, hsdR514 | |

Information on genotypes of strains from Keio collection can be found at https://cgsc.biology.yale.edu/StrainQueryForm.php

**Table 2: Oligonucleotide primers used (Sequences in Sequence Protocol)**

| 'KO' primers were used to amplify the Km knockout cassette from pKD4 with 50 bp gene-specific upstream and downstream sequences. To verify gene replacement by kanamycin resistance cassette and cassette removal by flippase, 'KO-Ver'-primers (knockout-verification) were used. Internal primers were used to verify successful removal of the gene from the genome. | | |
|---|---|---|
| **SEQ ID** | **name** | **purpose** |
| NO 1 | Ndh_KO_fwd | Cassette construction |
| NO 2 | Ndh_KO_rvs | Cassette construction |
| NO 3 | Ndh_KO_Ver_fwd | Deletion verification |
| NO 4 | Ndh_KO_Ver_rvs | Deletion verification |
| NO 5 | Ndh int fwd | Deletion verification |
| NO 6 | Ndh_int_rvs | Deletion verification |
| NO 7 | NuoEFG_KO_fwd | Cassette construction |
| NO 8 | NuoEFG _KO_rvs | Cassette construction |
| NO 9 | NuoEFG KO Ver fwd | Deletion verification |
| NO 10 | NuoEFG KO Ver rvs | Deletion verification |
| NO 11 | NuoEFG _int_fwd | Deletion verification |
| NO 12 | NuoEFG _int_rvs | Deletion verification |
| NO 13 | ubiCA_KO_fwd | Cassette construction |
| NO 14 | ubiCA KO rvs | Cassette construction |
| NO 15 | ubiCA_KO_Ver_fwd | Deletion verification |
| NO 16 | ubiCA_KO_Ver_rvs | Deletion verification |
| NO 17 | putA_KO Ver finid | Deletion verification |
| NO 18 | putA_KO_Ver_rvs | Deletion verification |
| NO 19 | putA int fwd | Deletion verification |
| NO 20 | putA_int_rvs | Deletion verification |
| NO 21 | glcDEF_KO_fwd | Cassette construction |
| NO 22 | glcDEF_KO_rvs | Cassette construction |
| NO 23 | glcDEF KO Ver fwd | Deletion verification |
| NO 24 | glcDEF KO Ver rvs | Deletion verification |
| NO 25 | gIcDEF_int finid | Deletion verification |
| NO 26 | glcDEF_int_rvs | Deletion verification |
| NO 27 | dld_KO_Ver_fwd | Deletion verification |
| NO 28 | *dId* KO_Ver_rvs | Deletion verification |
| NO 29 | dld_int_fwd | Deletion verification |
| NO 30 | dld_int_rvs | Deletion verification |
| 31 | mqo_KO_Ver_fwd | Deletion verification |
| NO 32 | mqo_KO_Ver_rvs | Deletion verification |
| NO 33 | mqo int fwd | Deletion verification |
| NO 34 | mqo_int_rvs | Deletion verification |
| NO 35 | dadA_KO_Ver_fwd | Deletion verification |
| NO 36 | dadA_KO_Ver_rvs | Deletion verification |
| NO 37 | dadA int fwd | Deletion verification |
| NO 38 | dadA int rvs | Deletion verification |
| NO 39 | poxB_KO Ver finid | Deletion verification |
| NO 40 | poxB_KO_Ver_rvs | Deletion verification |
| NO 41 | poxB_int finid | Deletion verification |
| NO 42 | poxB_int_rvs | Deletion verification |
| NO 43 | kefF_KO_Ver_fwd | Deletion verification |
| NO 44 | kefF_KO_Ver_rvs | Deletion verification |
| NO 45 | kefF_int_fwd | Deletion verification |
| NO 46 | kefF_int_rvs | Deletion verification |
| NO 47 | wrbA_KO_Ver_fwd | Deletion verification |
| NO 48 | wrbA_KO_Ver_rvs | Deletion verification |
| NO 49 | wrbA_int_fwd | Deletion verification |
| NO 50 | wrbA_int_rvs | Deletion verification |
| NO 51 | fadE_KO_Ver_fwd | Deletion verification |
| NO 52 | fadE_KO_Ver_rvs | Deletion verification |
| NO 53 | fadE_int_fwd | Deletion verification |
| NO 54 | fadE_int_rvs | Deletion verification |
| NO 55 | yieF_KO_Ver_fwd | Deletion verification |
| NO 56 | yieF_KO_Ver_rvs | Deletion verification |
| NO 57 | yieF_int_fwd | Deletion verification |
| NO 58 | yieF_int_rvs | Deletion verification |
| NO 59 | mdaB-ygiN_KO_C_R | Cassette construction |
| NO 60 | mdaB-ygiN_KO_V_F | Cassette construction |
| NO 61 | mdaB-ygiN_KO_Ver_fwd | Deletion verification |
| NO 62 | mdaB-ygiN_KO_Ver_rvs | Deletion verification |
| NO 63 | mdaB-ygiN_int_fwd | Deletion verification |
| NO 64 | mdaB-ygiN_int_rvs | Deletion verification |
| NO 65 | glpD_KO_Ver_fwd | Deletion verification |
| NO 66 | glpD_KO_Ver_rvs | Deletion verification |
| NO 67 | glpD_int_fwd | Deletion verification |
| NO 68 | glpD_int_rvs | Deletion verification |
| NO 69 | lldD_KO_Ver_fwd | Deletion verification |
| NO 70 | lldD_KO_Ver_rvs | Deletion verification |
| NO 71 | lldD_int_fwd | Deletion verification |
| NO 72 | lldD_int_rvs | Deletion verification |
| NO 73 | glpABC_KO_C_F | Cassette construction |
| NO 74 | glpABC_KO_C_R | Cassette construction |
| NO 75 | glpABC_KO_Ver_fwd | Deletion verification |
| NO 76 | glpABC_KO_ Ver_rvs | Deletion verification |
| NO 77 | glpABC_int_fwd | Deletion verification |
| NO 78 | glpABC_int_rvs | Deletion verification |
| NO 79 | sdhABCD_KO_C_F | Cassette construction |
| NO 80 | sdhABCD_KO_C_R | Cassette construction |
| NO 81 | sdhABCD_KO_Ver_fwd | Deletion verification |
| NO 82 | sdhABCD_KO_Ver_rvs | Deletion verification |
| NO 83 | sdhABCD_int_fwd | Deletion verification |
| NO 84 | sdhABCD_int_rvs | Deletion verification |
| NO 85 | IhgO_KO_Ver_fwd | Deletion verification |
| NO 86 | IhgO_KO_Ver_rvs | Deletion verification |
| NO 87 | IhgO_int_fwd | Deletion verification |
| NO 88 | IhgO_int_rvs | Deletion verification |
| NO 89 | gpsA_KO_F | Cassette construction |
| NO 90 | gpsA_KO_R | Cassette construction |
| NO 91 | gpsA_KO_V_F | Deletion verification |
| NO 92 | gpsA_KO_V_R | Deletion verification |
| NO 93 | gpsA fwd internal | Deletion verification |
| NO 94 | gpsA internal Rev | Deletion verification |

**Table 3: List of deleted genes**

| Gr. | Abbreviation | Encoded protein | Gene locus |
|---|---|---|---|
| D | *dadA* | D-amino acid dehydrogenase | b1189 |
| D | *dld* | quinone-dependent D-lactate dehydrogenase | b2133 |
| D | *fadE* | Acyl-coenzyme A dehydrogenase | b0221 |
| D | *glcDEF, or glcD, glcE, glcF* | glycolate oxidase subunits D, E, and F | D: b2979; E: b4468; F: b4467, |
| D | *glpABC or glpA, glpB, glpC* | Anaerobic glycerol-3-phosphate dehydrogenase subunit A, B, C | A: b2241; B: b2242; C: b2243, |
| D | *glpD* | glycerol-3-phosphate dehydrogenase | b3426 |
| D | *gpsA* | Glycerol-3-phosphate dehydrogenase [NAD(P)⁺] (GPDA) | b3608 |
| B | *kefF* | regulator of KefC-mediated potassium transport and quinone oxidoreductase | b0046 |
| D | *lhgO* | L-2-hydroxyglutarate dehydrogenase | b2660 |
| D | *lldD* | L-lactate dehydrogenase | b3605 |
| B | *mdaB* | NADPH:quinone oxidoreductase MdaB | b3028 |
| D | *mqo* | malate:quinone oxidoreductase | b2210 |
| A | *ndh* | enzyme NADH:quinone oxidoreductase II | b1109 |
| A | *nuoABCDEFGHIJKL MN* or *nuoA,nuoB,nuoCD, nuoE, nuoF, nuoG,nuoH, nuol, nuoJ, nuoK, nuoL, nuoM* | encode the 13- 14 subunits of the protein complex NADH:ubiquinone oxidoreductase I, NDH-1: A, B, C/D, E, F, G, H, I, J, K, L, M | A:b2288, B:b2287, CD:b2286, E: b2285, F: b2284, G: b2283, H: b2282, I: b2281, J: b2280, K: b2279, L: b2278, M: b2277 |
| C | *poxB* | Pyruvate dehydrogenase [ubiquinone] | b0871 |
| D | *putA* | used DNA-binding transcriptional repressor / proline dehydrogenase / 1-pyrroline-5-carboxylate dehydrogenase PutA | b1014 |
| C | *sdhABCD or sdhA, sdhB, sdhC, sdhD* | Succinate dehydrogenase flavoprotein subunit A, B, C, or D | A: b0723; B: b0724; C: b0721; D: b0722 |
| B | *wrbA* | NAD(P)H:quinone oxidoreductase | b1004 |
| B | *ygiN* | quinol monooxygenase YgiN | b3029 |
| B | *yieF* | quinone reductase | b3713 |

| | | | |
|---|---|---|---|
| Group A: NDH activity, group B: described NDH activity, group C: quinone reducing reactions from central metabolism; group D: NDH bypassing activities. | | | |

## Claims

1. A genetically engineered bacteria comprising:
a deletion of *ndh* gene;
a deletion of one or more *nuo* genes;
a deletion of *dld* gene,
wherein the one or more *nuo* genes are selected from *nuoA, nuoB, nuoC, nuoD, nuoE, nuoF, nuoG, nuoH, nuoI, nuoJ, nuoK, nuoL, nuoM* and *nuoN.*

2. The genetically engineered bacteria according to claim 1 further comprising:
a deletion of *mqo* gene.

3. The genetically engineered bacteria according to claim 1 or 2 further comprising:
a deletion of *glpD* gene.

4. The genetically engineered bacteria according to any one of the claims 1 - 3 further comprising:
a deletion of *poxB* gene and/or a deletion of *sdhABCD* genes.

5. The genetically engineered bacteria according to claim any one of the claims 1 - 4 further comprising:
a deletion of one or more genes selected from the group comprising f *glcDEF* genes, *of lhgO* gene,of *putA* gene, of *lldD* gene and/or of *dadA* gene.

6. The genetically engineered bacteria according to any one of the claims 1 - 5 further comprising: a deletion of one or more genes selected from the group comprising *kefF* gene, *mdaB-ygiN* genes, *glpABC* genes, *gpsA* gene, *wrbA* gene, *yieF* gene, and *fadE* gene.

7. The genetically engineered bacteria according to any one of the claims 1 -6, wherein the one or more *nuo* genes are selected from *nuoE, nuoF* and/or *nuoG;* and preferably wherein bacteria comprises a deletion of *nuoE,* of *nuoF,* and of *nuoG* genes.

8. The genetically engineered bacteria according to any one of the claims 1 - 7, wherein the genetically engineered bacteria is a facultativ anaerob bacteria selected from the group comprising *Enterobacteriaceae, Staphylococcaceae, Streptococcaceae, Lactobacillaceae, Vibrionaceae,* and *Pasteurellaceae.*

9. The genetically engineered bacteria according to any one of the claims 1 - 8, wherein the genetically engineered bacteria is an *E. coli* or wherein the genetically engineered bacteria is an *E. coli* comprising a deletion of *ndh* gene, of *nuoEFG* genes, of *dld* gene, and optionally a deletion of *mqo* gene and/or of *glpD* gene.

10. The genetically engineered bacteria according to any one of the claims 1 - 9, wherein the genetically engineered bacteria is an *E. coli* comprising a deletion of *ndh* gene, of *nuoEFG* genes, of *dld* gene, of *mqo* gene, *poxB* gene and *sdhABCD* genes; and further comprising a deletion of *glcDEF* genes, *of IhgO* gene, of *putA* gene, of *lldD* gene, *of dadA* gene, of *kefF* gene, *of mdaB-ygiN* gene, of *gIpABC* genes, of *gpsA* gene, of *wrbA* gene, *of yieF* gene, *of fadE* gene, and optionally of *glpD.*

11. The genetically engineered bacteria according to any one of the claims 1 - 10, wherein the genetically engineered bacteria is an *E. coli* MG1655 or an *E. coli* MG1655 further comprising one or more recombination systems selected from the group comprising lambda Red recombinases, Rec recombinases, and flippase recombinase.

12. A method for producing D-lactate comprising the following steps:
a) providing the genetically engineered bacteria of any one of the claims 1 - 11 ;
b) providing a culture medium comprising a carbon source;
c) culturing the genetically engineered bacteria under aerobic conditions in the culture medium of b) to produce a culture medium comprising D-lactate,
wherein the carbon source is selected from the group comprising glucose, fructose, xylose, arabinose, galactose, mannose, sucrose, cellobiose, hemicellulose, and glycerol or a combination thereof.

13. The method according to claim 12, wherein the genetically engineered bacteria comprises a *glpD* deletion and
wherein the carbon source is is selected from the group comprising glucose, fructose, xylose, arabinose, galactose, mannose, sucrose, cellobiose, and hemicellulose, or a combination thereof.

14. The method according to claim 12 or 13, wherein the genetically engineered bacteria does not comprise a *glpO* deletion and
wherein the carbon source is glycerol.

15. The method according to any one of the claims 12 - 14, wherein the genetically engineered bacteria is a facultativ anaerob bacteria selected from the group comprising *Enterobacteriaceae, Staphylococcaceae, Streptococcaceae, Lactobacillaceae, Vibrionaceae,* and *Pasteurellaceae* or wherein the genetically engineered bacteria is an *E. coli.*
